(19) Europäisches Patentamt / European Patent Office / Office européen des brevets

(11) **EP 3 276 340 A1**

(12) **EUROPEAN PATENT APPLICATION**

(43) Date of publication:
**31.01.2018 Bulletin 2018/05**

(51) Int Cl.:
***G01N 27/327*** (2006.01)

(21) Application number: **17183246.2**

(22) Date of filing: **26.07.2017**

| | |
|---|---|
| (84) Designated Contracting States:<br>**AL AT BE BG CH CY CZ DE DK EE ES FI FR GB GR HR HU IE IS IT LI LT LU LV MC MK MT NL NO PL PT RO RS SE SI SK SM TR**<br>Designated Extension States:<br>**BA ME**<br>Designated Validation States:<br>**MA MD**<br><br>(30) Priority: **27.07.2016 JP 2016147709**<br><br>(71) Applicant: **SYSMEX CORPORATION**<br>**Kobe-shi**<br>**Hyogo 651-0073 (JP)** | (72) Inventors:<br>• **Hori, Nobuyasu**<br>**Hyogo, 651-0073 (JP)**<br>• **Kojima, Junko**<br>**Hyogo, 651-0073 (JP)**<br>• **Morishita, Yu**<br>**Hyogo, 651-0073 (JP)**<br><br>(74) Representative: **Hoffmann Eitle**<br>**Patent- und Rechtsanwälte PartmbB**<br>**Arabellastraße 30**<br>**81925 München (DE)** |

(54) **ELECTRODE AND MANUFACTURING METHOD THEREOF, ENZYME SENSOR, GLUCOSE SENSOR AND IN-VIVO COMPONENT MEASURING DEVICE**

(57)     In a hydrogen peroxide electrode 100 used as a working electrode 30 of an enzyme sensor 20, an intermediate layer 101 containing particles and a retentive substance for holding the particles are provided on a substrate 21, and an electrode layer 107 is provided on the intermediate layer 101. In this way, according to the hydrogen peroxide electrode 100, hydrogen peroxide can be detected with high sensitivity even when an applied voltage that is not susceptible to interference by interference substances present in the living body is used.

FIG. 1

**Description**

FIELD OF THE INVENTION

The present invention relates to an electrode for detecting hydrogen peroxide, a method for producing same, an enzyme sensor, a glucose sensor and an in-vivo component measuring device.

BACKGROUND

In-vivo components contained in bodily fluids such as blood and urine, for example, glucose and uric acid, are indicators of diabetes and gout, respectively. In order to detect such diseases at an early stage, it is required to be able to accurately detect in vivo components.

Japanese Patent Application Publication No. 63-241347 discloses a technique for detecting glucose in a body fluid using an enzyme sensor having a hydrogen peroxide electrode for detecting hydrogen peroxide. As shown in FIG. 15, in the enzyme sensor 500 described in Patent Application Publication: No. 63-241347, a conductive thin film electrode layer 502 is formed on a substrate 501, and a hydrogen peroxide selective permeation film 503 is provided on the surface of the conductive thin film electrode layer 502. An enzyme immobilization layer 504 having glucose oxidase immobilized thereon is provided on the surface of the hydrogen peroxide selective permeable membrane 503. Glucose in the body fluid reacts with oxygen in the presence of glucose oxidase immobilized on the enzyme immobilization layer 504 to generate hydrogen peroxide. The generated hydrogen peroxide permeates through the hydrogen peroxide selective permeable membrane 503 and is electrolyzed on the conductive thin film electrode layer 502 to generate a current. The amount of glucose is quantified by measuring this current value.

In the case of quantifying the amount of glucose using such an enzyme sensor, it is known that when a voltage necessary for electrolysis of hydrogen peroxide is applied, a reducing substance such as ascorbic acid contained in the body fluid is also electrolyzed in the electrode layer. An electric current is generated when ascorbic acid or the like is electrolyzed on the electrode layer, which makes it difficult to accurately measure the current value attributable only to hydrogen peroxide, and there is a possibility that the glucose detection accuracy may be reduced. Therefore, in order to suppress the electrolysis of interfering substances such as ascorbic acid in the conductive thin film electrode layer 502 in the enzyme sensor 500 of Patent Application Publication: No. 63-241347, a hydrogen peroxide selective permeation film 503 is provided on the surface of the conductive thin film electrode layer 502 so that only hydrogen peroxide generated by the enzymatic reaction is selectively transmitted to the conductive thin film electrode layer 502 side.

SUMMARY OF THE INVENTION

However, in the case of using the enzyme sensor 500 described in Patent Application Publication: No. 63-241347, it is difficult to accurately quantify the amount of glucose by the current value since it is difficult for the hydrogen peroxide selective permeation membrane 503 to transmit all hydrogen peroxide generated from glucose and only part of the hydrogen peroxide passes through the hydrogen peroxide selective permeable membrane 503.

It is desirable to suppress the influence of interfering substances and to detect hydrogen peroxide with higher sensitivity.

An electrode according to a first aspect of the present invention incorporates: an intermediate layer being disposed on a substrate and including particles and a retentive substance for holding the particles; and an electrode layer being disposed on the intermediate layer and detecting hydrogen peroxide. The inventors of the present invention found that the influence of interfering substances can be suppressed and hydrogen peroxide can be detected with high sensitivity in the electrode layer by providing an intermediate layer containing particles and a retentive substance between the substrate and the electrode layer without providing a hydrogen peroxide selective permeation film on the electrode layer. Specifically, the present inventors have found that hydrogen peroxide is electrolyzed in the electrode layer and the generated current can be detected with high sensitivity even when using a low applied voltage such that an interfering substance present in the living body is not electrolyzed in the electrode layer. Therefore, in the electrode according to the first aspect of the present invention, it is possible to detect a current caused only by hydrogen peroxide by using a low applied voltage such that an interfering substance is not electrolyzed in the electrode layer, and it is possible to suppress a reduction of the amount of hydrogen peroxide reaching the electrode layer even without providing a hydrogen peroxide selective permeation film on the layer. Therefore, according to the electrode according to the first aspect of the present invention, it is possible to detect hydrogen peroxide with higher sensitivity by suppressing the influence of interfering substances. Note that, in the present specification, the term "particles" refers to particles in a cured state such as microspheres, nanoparticles, microparticles or beads. "Particles" also may be particles in a semi-cured state. "Retentive substance" refers to a substance capable of dispersing and holding particles between a substrate and an electrode layer. The "retentive substance" may be a hydrophilic polymer substance, a hydrophobic polymer substance or the like.

"Intermediate layer" refers to a layer disposed on a substrate and provided between a substrate and an electrode layer. An adhesive layer for adhering the electrode layer may be provided on the surface of the intermediate layer. "Electrode layer" is a layer formed of a thin metal film such as gold or platinum. The electrode layer has the function of electrolyzing hydrogen peroxide and receiving generated electrons.

It is preferable that a layer containing particles and a layer containing a retentive substance are laminated in the intermediate layer. The present inventors have found that the strength of the hydrogen peroxide electrode is improved in this case.

The particles also may be nanoparticles or microparticles. The particles also may be porous particles. The shape of the particle also may be a spherical shape or a non-spherical shape. The non-spherical shape includes a columnar shape, a prismatic shape, a plate shape and the like.

The retentive substance may be a hydrophilic polymeric substance. In the present specification, the term "hydrophilic polymeric substance" is a water-soluble polymer compound or a polymer compound interacting with water molecules by electrostatic interaction or hydrogen bonding even if it is water-insoluble. The hydrophilic polymer substance is preferably polyvinyl alcohol. Polyvinyl alcohol shows high adhesion to particles. Therefore, the strength of the hydrogen peroxide electrode can be improved insofar as the hydrogen peroxide electrode has such a configuration.

An enzyme sensor according to a second aspect of the present invention incorporates a substrate, an electrode disposed on the substrate, and an oxidase immobilized on the surface of the electrode. The enzyme sensor is used for the measurement of substances that produce hydrogen peroxide as a product by the action of the enzyme oxidase. According to the enzyme sensor of the second aspect of the present invention, since the above-described electrode is provided, a current caused by hydrogen peroxide can detect with high sensitivity even when an applied voltage that is not susceptible to interference by interference substances present in the living body is used. Therefore, according to the enzyme sensor of the second aspect of the present invention, the in vivo component that produces hydrogen peroxide as a product by the action of oxidase can be detected with high sensitivity.

A glucose sensor according to a third aspect of the present invention incorporates a substrate, an electrode disposed on the substrate, and glucose oxidase immobilized on the surface of the electrode. The glucose sensor is used for measuring glucose based on hydrogen peroxide caused by glucose. According to the glucose sensor of the third aspect of the present invention, since the above-described electrodes are provided, a current caused by hydrogen peroxide can detect with high sensitivity even when an applied voltage that is not susceptible to interference by interference substances present in the living body is used. Therefore, according to the glucose sensor of the third aspect of the present invention, it is possible to suppress the influence of interfering substances and to detect glucose with high sensitivity.

The in-vivo component measuring device according to a fourth aspect of the present invention incorporates a detection unit for obtaining component information relating to the amount of a biological component of a measurement target contained in a body fluid extracted from a living body, and an analysis unit for acquiring an analysis value relating to the amount of the in-vivo component of the measurement target based on the component information, wherein the detection unit includes a working electrode which includes the aforesaid electrode and oxidase immobilized on the surface thereof, and a counter electrode. The in-vivo component measuring device is used for measuring in-vivo components that produce hydrogen peroxide as a product through the action of oxidase. The in-vivo component measuring apparatus according to the fourth aspect of the present invention incorporates the working electrode including the above-described electrode, so that it is possible, with high sensitivity, to detect a current caused by hydrogen peroxide generated from an in-vivo component. Therefore, the in-vivo component measuring device according to the fourth aspect of the present invention, it is possible to suppress the influence of interfering substances and to detect the in-vivo component with high sensitivity.

The electrode manufacturing method according to a fifth aspect of the present invention, includes the steps of: (A) forming an intermediate layer containing particles and a retentive substance for holding the particles on one surface of a substrate, and (B) a step of forming an electrode layer on the surface of the intermediate layer. The electrode manufacturing method according to the fifth aspect of the present invention can obtain an electrode possessing the aforesaid action effect by performing an operation of forming an intermediate layer on a substrate and forming an electrode layer on the surface of the formed intermediate layer.

In step (A), it is preferable to form a layer containing particles after forming a layer containing a retentive substance on one surface of the substrate. In this case, an electrode with better strength can be obtained.

According to the present invention, it is possible to provide a novel means and an application thereof which can suppress the influence of interfering substances and detect hydrogen peroxide with higher sensitivity.

BRIEF DESCRIPTION OF THE DRAWINGS

FIG. 1 is a schematic cross sectional view showing a hydrogen peroxide electrode;

FIG. 2 is a schematic cross sectional view showing a hydrogen peroxide electrode;

FIG. 3 is a schematic plan view showing an enzyme sensor including a hydrogen peroxide electrode;

FIG. 4 is a perspective view showing an example of an in-vivo component measuring device;

FIG. 5 is a schematic plan view showing an in-vivo component measuring device;

FIG. 6 is a schematic side view showing an in-vivo component measuring device;

FIG. 7 is a process chart showing a procedure for manufacturing a hydrogen peroxide electrode;

FIG. 8 (A) is a graph showing the relationship between the detection sensitivity of glucose and the applied voltage when the glucose sensor of example 1 is used, (B) is a graph showing the relationship between the detection sensitivity of acetaminophen and the applied voltage when the glucose sensor of example 1 is used;

FIG. 9 (A) is a graph showing the relationship between the detection sensitivity of glucose and the applied voltage when the glucose sensor of comparative example 1 is used, (B) a graph showing the relationship between the detection voltage of acetaminophen and the applied voltage when the glucose sensor of comparative example 1 is used;

FIG. 10 (A) is a graph showing the relationship between the detection sensitivity of glucose and the applied voltage when the glucose sensor of comparative example 2 is used, (B) is a graph showing the relationship between the interference rate due to acetaminophen and the applied voltage when the glucose sensor of comparative example 2 is used, and (C) is a graph showing the relationship between the interference rate of due to ascorbic acid and the applied voltage when the glucose sensor of comparative example 2 is used;

FIG. 11 (A) is a graph showing the relationship between the detection sensitivity of glucose and the applied voltage when glucose sensors of example 2 and comparative example 2 are used, and (B) is a graph showing the relationship between the interference rate by acetaminophen and the applied voltage when the glucose sensor of example 2 and comparative example 2 are used;

FIG. 12 (A) is a graph showing the relationship between the detection sensitivity of glucose and the applied voltage when the glucose sensors of example 3 and comparative example 2 are used, (B) is a graph showing the relationship between the interference rate due to acetaminophen and the applied voltage when the glucose sensors of example 2 and comparative example 2 are used, and (C) is a graph showing the relationship between the interference rate due to ascorbic acid and the applied voltage when glucose sensors of example 3 and comparative example 2 are used;

FIG. 13 (A) is a graph showing the relationship between the detection sensitivity of glucose and the applied voltage when glucose sensors of example 4 and comparative example 2 are used, (B) is a graph showing the relationship between the interference rate due to acetaminophen when the glucose sensor of example 4 and comparative example 2 are used, and (C) is a graph showing the relationship between the interference rate due to ascorbic acid and the applied voltage when the glucose sensors of example 4 and comparative example 2 are used;

FIG. 14 (A) is a drawing substitute photograph of the surface of the working electrode of the electrode substrate of Experiment 1, (B) is a drawing substitute photograph of the surface of the working electrode of the electrode substrate of experiment 2, and (C) is a drawing substitute photograph of the surface of the working electrode of the electrode substrate obtained in example 2 (3); and

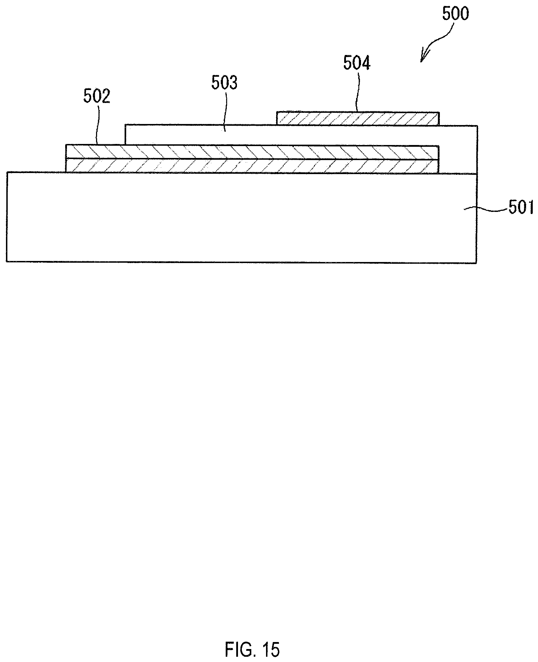

FIG. 15 is a schematic cross sectional view of a conventional enzyme sensor.

DESCRIPTION OF THE EMBODIMENTS OF THE INVENTION

In this specification, the description of numerical ranges by endpoints such as "X to Y" includes all numbers and rational numbers included in each range as well as the endpoints described therein.

"Microinvasive interstitial fluid extraction technique" refers to a technique of extracting interstitial fluid from a subject with slight invasion. In microinvasive interstitial fluid extraction technology, micropores are formed in the skin of the living body by a puncture tool or the like to promote extraction of interstitial fluid. The tissue fluid extracted from the micropores is collected using an extraction medium such as a gel.

"In vivo component" refers to a component contained in a living body, for example, body fluid extracted from a subject. Examples of in vivo components include, but are not limited to, glucose, uric acid, lactic acid, galactose and the like. Interstitial fluid, blood and the like are examples of body fluids, but there is no particular limitation.

Hydrogen Peroxide Electrode

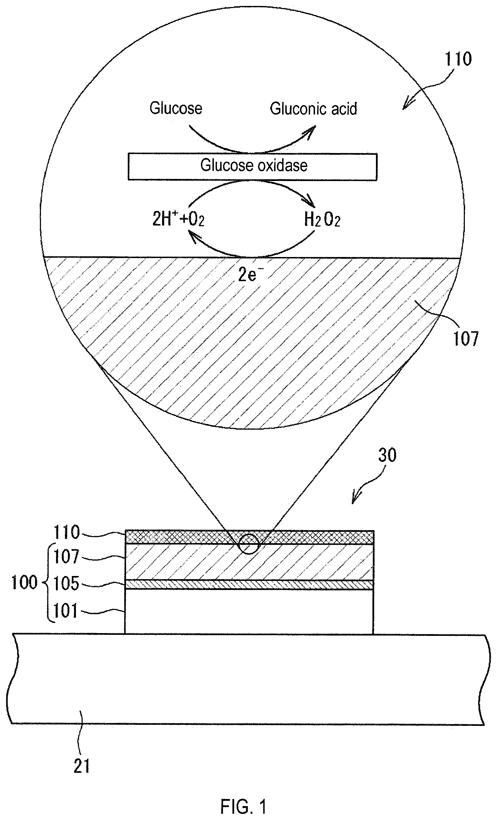

The hydrogen peroxide electrode is described with reference to the drawings. As shown in FIG. 1, the hydrogen peroxide electrode 100 is provided on a substrate 21. The hydrogen peroxide electrode 100 includes an intermediate layer 101, an adhesive layer 105, and an electrode layer 107. The hydrogen peroxide electrode 100 can be used as an electrode for detecting hydrogen peroxide generated from an in-vivo component in the presence of oxidase by, for example, immobilizing oxidase on the surface of the electrode layer 107, and bringing a gel containing oxidase or the like into contact with the surface of the electrode layer 107. When bringing the gel into contact with the surface of the electrode layer 107, it is possible to detect hydrogen peroxide generated from in-vivo components extracted to the gel by the hydrogen peroxide electrode 100.

The intermediate layer 101 is provided on the substrate 21. The electrode 107 is provided on the surface of the intermediate layer 101 through an adhesive layer 105. The intermediate layer 101 contains particles and a hydrophilic polymeric substance as a retentive substance.

The present inventors fabricated a hydrogen peroxide electrode 100 including an intermediate layer 101 between a substrate 21 and an electrode layer 107, and found that hydrogen peroxide can be detected with high sensitivity even when using a low applied voltage that is unlikely to electrolyze interfering substances present in the body by the electrode layer 107. This effect will be described later with examples.

Examples of the hydrophilic polymer substance include polyvinyl alcohol, carboxymethyl cellulose, methyl cellulose, cellulose ether, polyethylene oxide, polypropylene oxide, polyvinyl pyrrolidone, sodium polyacrylate, starch, polyacrylamide, derivatives thereof and the like, but are not particularly limited. Among these hydrophilic polymeric substances, polyvinyl alcohol is preferable because of high adhesion of particles. In this case, the strength of the hydrogen peroxide electrode can be improved.

The shape of the particle also may be a spherical shape or a non-spherical shape. The non-spherical shape includes a columnar shape, a prismatic shape, a plate shape and the like. Examples of particles include, for example, nanoparticles, microparticles, particles made of a porous material, and the like, but are not particularly limited; "nanoparticle" usually refers to a particle having an average particle diameter of 1 to 500 nm. "Microparticle" refers to a particle having an average particle size of 0.5 to 100 $\mu$m. In the present specification, the average particle size refers to a value directly observed using an electron microscope and measured using a reference scale built in the electron microscope. Specific examples of the particles include polystyrene beads, acrylic beads, silicon dioxide particles, alumina particles, zinc oxide particles, iridium oxide particles, indium oxide (In203) particles, titanium dioxide particles, mesoporous silica, mesoporous aluminosilicate, mixtures thereof, but are not particularly limited. The particles are preferably non-conductive particles. The average diameter of the particles is preferably 0.001 $\mu$m or more, more preferably 0.005 $\mu$m or more, and most preferably 0.01 $\mu$m or more from the viewpoint of detecting glucose with high sensitivity without being affected by interfering substances, and is preferably 10 $\mu$m or less, more preferably 5 $\mu$m or less, and most preferably 2 $\mu$m or less from the same perspective.

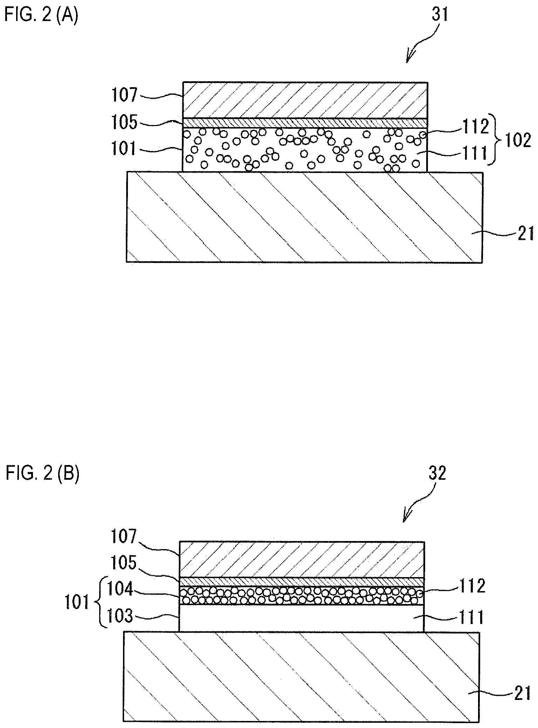

The intermediate layer 101 may have a single-layer structure as shown in FIG. 2 (A) or may have a two-layer structure as shown in FIG. 2 (B). The single layer 102 shown in FIG. 2 (A) has a structure in which particles 112 are dispersed in a hydrophilic polymer substance 111. The two-layer structure shown in FIG. 2 (B) is configured by a first intermediate layer 103 containing a hydrophilic polymer substance 111 and a second intermediate layer 104 provided on the first intermediate layer 103 and including particles 112. From the viewpoint of improving the strength of the hydrogen peroxide electrode 100, the intermediate layer 101 preferably has a two-layer structure as shown in FIG. 2 (B).

The thickness of the intermediate layer 101 is not particularly limited insofar as it does not affect the conductivity of the electrode layer 107. In general, the thickness of the intermediate layer 101 is preferably 0.001 $\mu$m or more, more preferably 0.01 $\mu$m or more from the viewpoint of reliably adhering the particles on the substrate, and is preferably 100 $\mu$m or less, more preferably 50 $\mu$m or less, and most preferably 30 $\mu$m or less from the viewpoint of suppressing peeling of the electrode layer from the substrate.

In the case where the intermediate layer 101 has a two-layer structure, the thickness of the first intermediate layer 103 and the thickness of the second intermediate layer 104 may be within the range of the thickness of the intermediate layer 101. In this case, the thickness of the first intermediate layer 103 usually is preferably 0.001 $\mu$m or more, and more preferably 0.01 $\mu$m or more from the viewpoint of particle adhesion, and preferably 1000 $\mu$m or less, and more preferably 100 $\mu$m or less from the viewpoint of peeling from the substrate. In the second intermediate layer 104, the particles may be stacked in the thickness direction of the second intermediate layer 104, or may not be stacked. In the case where particles are not stacked in the thickness direction of the second intermediate layer 104, the thickness of the second intermediate layer 104 is usually about the same as the particle diameter of the particles. In the case where particles are stacked in the thickness direction of the second intermediate layer 104, the thickness of the second intermediate layer 104 is usually about the same as the thickness obtained by multiplying the particle diameter by the number of particles stacked in the thickness direction. thickness

In general, the density of the particles 112 in the intermediate layer 101 is preferably $9 \times 10^4$/mm$^2$ or more, more preferably $2 \times 10^5$/mm$^2$ or more, from the viewpoint of highly sensitive detection of hydrogen peroxide, From the viewpoint of reduction, it is preferably $9 \times 10^9$ particles/mm$^2$ or less, more preferably $1 \times 10^9$ particles/mm$^2$ or less.

The adhesive layer 105 is a layer for adhering the intermediate layer 101 and the electrode layer 107. Examples of material configuring the adhesive layer 105, for example, include titanium and the like, but are not particularly limited. The thickness of the adhesive layer 105 may be any thickness insofar as it is sufficient to adhere the intermediate layer 101 and the electrode layer 107. The thickness of the adhesive layer 105 is preferably 0.001 $\mu$m or more, and more preferably 0.005 $\mu$m or more from the viewpoint of firmly adhering the electrode layer onto the substrate, and preferably

0.03 μm or less, and more preferably 0.02 μm or less from the viewpoint of securing adhesiveness and reducing manufacturing cost.

The electrode layer 107 exchanges electrons with hydrogen peroxide caused by glucose. The thickness of the electrode layer 107 is not particularly limited insofar as the thickness maintains conductivity. The thickness of the electrode layer 107 is preferably 0.01 μm or more, and more preferably 0.05 μm or more from the viewpoint of conductivity, and is preferably 0.5 μm or less, more preferably 0 .3 μm or less from the viewpoint of manufacturing cost.

In the present embodiment, the shape of the hydrogen peroxide electrode 100 is circular. The shape of the hydrogen peroxide electrode 100 also may be a shape other than a circular shape. The shape of the hydrogen peroxide electrode 100 may be, for example, a rectangular shape, a polygonal shape or the like, but is not particularly limited.

Enzyme Sensor Structure

The aforementioned hydrogen peroxide electrode 100 can be used as an electrode of an enzyme sensor for detecting a substance which produces hydrogen peroxide as an enzyme product by the action of oxidase which is an enzyme. The enzyme sensor is described with reference to the drawings. In the following description, a glucose sensor used for detection of glucose contained in interstitial fluid extracted from a subject will be described as an example.

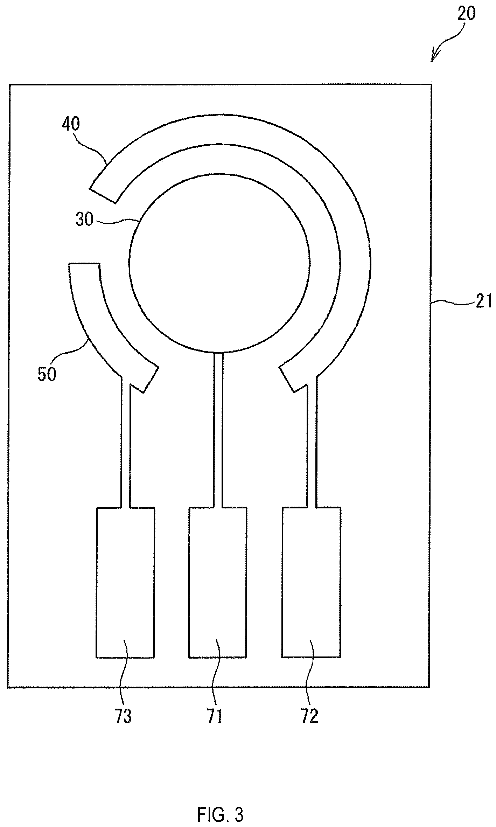

As shown in FIG. 3, the enzyme sensor 20 includes a substrate 21, a working electrode 30, a counter electrode 40, a reference electrode 50, and electrode leads 71, 72, 73. The working electrode 30, the counter electrode 40, the reference electrode 50, and the electrode leads 71, 72, 73 are provided on one surface of the substrate 21.

The substrate 21 has a rectangular shape. Note that the shape of the substrate 21 may be a polygonal shape, a circular shape, or the like, and is not particularly limited.

The material configuring the substrate 21 is an insulating material which does not affect at least the conductivity of the working electrode 30, the counter electrode 40 and the reference electrode 50. Examples of the material configuring the substrate 21 include polyester resins such as polyvinyl alcohol, polyethylene terephthalate, polybutylene terephthalate, polyethylene naphthalate, and polybutylene naphthalate, as well as polyimide, glass epoxy resin, glass, ceramic and the like, and the material is not particularly limited. The thickness of the substrate 21 can be appropriately determined according to the use of the enzyme sensor or the like.

As shown in FIG. 3, the working electrode 30 is disposed on one side of the surface of the substrate 21. The working electrode 30 is connected to the electrode lead 71. The electrode lead 71 extends from the working electrode 30 toward the other side part of the substrate 21, more specifically, toward the lower side of the substrate 21 in FIG. 3.

Returning to FIG. 1, the working electrode 30 includes a hydrogen peroxide electrode 100 formed on one surface of the substrate 21, and an enzyme layer 110 containing glucose oxidase. The enzyme layer 110 is provided on the surface of the hydrogen peroxide electrode 100.

The enzyme layer 110 is provided on the surface of the electrode layer 107 in the hydrogen peroxide electrode 100. In this embodiment, the enzyme layer 110 includes glucose oxidase. Glucose oxidase is an enzyme that generates hydrogen peroxide using glucose as a substrate. The organism which is the source of glucose oxidase is not particularly limited. The amount of glucose oxidase in the enzyme layer 110, when converted into a glucose oxidase GOD active amount in the solution applied on the electrode layer, is preferably 0.01 U / μL or more, and more preferably 0.1 U / μL or more from the viewpoint of the detection sensitivity of glucose. From the viewpoint of cost, the amount is preferably 100 U/μL or less, and more preferably 50 U/μL or less.

The working electrode 30 is brought into contact with bodily fluid. When glucose is contained in the body fluid, as shown in FIG. 1, glucose oxidase existing in the enzyme layer 110 in the working electrode 30 oxidizes the glucose. Oxidation of glucose produces hydrogen peroxide and gluconic acid. The generated hydrogen peroxide is electrolyzed on the electrode layer 107 of the hydrogen peroxide electrode 100 of the working electrode 30. The electrode layer 107 contains a conductive material. A metal material such as platinum, gold and the like can be the conductive material used for the electrode layer 107, but the material is not particularly limited. According to the enzyme sensor 20, glucose contained in a body fluid can be detected based on the current generated through electrolysis of hydrogen peroxide.

The counter electrode 40 is arranged outside the working electrode 30, more specifically, on the surface of the substrate 21 on the upper side of the working electrode 30 in FIG. 3. The counter electrode 40 is connected to the electrode lead 72. The electrode lead 72 extends from the counter electrode 40 toward the other side part of the substrate 21, more specifically, toward the lower side of the substrate 21 in FIG. 3. The counter electrode 40 includes an electrode layer containing a conductive material. A metal material such as platinum, gold and the like can be the conductive material used for the electrode layer in the counter electrode 40, but the material is not particularly limited. <t0/> The thickness of the electrode layer in the counter electrode 40 can be appropriately determined depending on the use of the enzyme sensor or the like. Note that the counter electrode 40 may have the same configuration as the hydrogen peroxide electrode 100.

In FIG. 3, the reference electrode 50 is disposed at a position facing the counter electrode 40 across the working electrode 30. The reference electrode 50 is connected to the electrode lead 73. The electrode lead 73 extends from the reference electrode 50 toward the other side part of the substrate 21, more specifically, toward the lower side of the substrate 21 in FIG. 3. The reference electrode 50 includes an electrode layer containing a conductive material. Examples of the conductive material include, for example, metals such as platinum, gold, silver, copper, carbon, palladium, chromium, aluminum, nickel, alloys including at least one of these metals, metal halogens such as chlorides of these metals and the like, but are not particularly limited. Specific examples of the reference electrode include a silver-silver chloride electrode and the like, but are not particularly limited. The thickness of the electrode layer in the reference electrode 50 can be appropriately decided according to the use of the measuring device 10 or the like. The enzyme sensor 20 also may omit the reference electrode 50. In this case, depending on the type of the conductive material used for the counter electrode 40 and the thickness of the counter electrode 40 and the like, the counter electrode 40 also may serve as the reference electrode 50. When measuring a large current, the enzyme sensor 20 preferably includes the reference electrode 50 from the viewpoint of suppressing the influence of the voltage drop and stabilizing the voltage applied to the working electrode 30.

The electrode lead 71, the electrode lead 72, and the electrode lead 73 are arranged so as to be parallel to each other at the other side portion of the substrate 21, more specifically, at the lower side portion of the substrate 21 in FIG. 3.

Enzyme Sensor Modifications

Instead of glucose oxidase contained in the enzyme layer 110 of the working electrode 30 in the enzyme sensor 20, another enzyme which generates hydrogen peroxide by action on a substance serving as a substrate can also be used. Substances other than glucose can be detected depending on the enzyme sensor 20 including other enzymes. Examples of such enzymes include, but are not limited to, lactate oxidase, galactose oxidase, cholesterol oxidase and the like.

In-Vivo Component Measuring Device

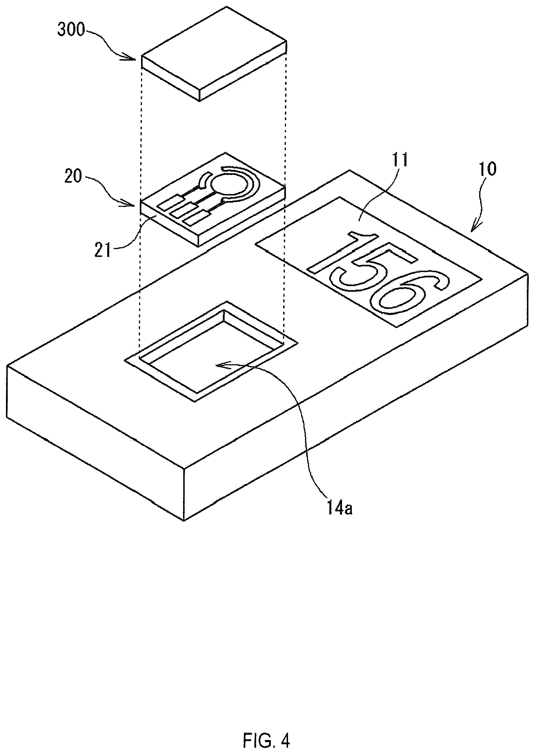

The enzyme sensor 20 including the above hydrogen peroxide electrode 100 can be used as an enzyme sensor of an in-vivo component measuring device for measuring the amount of a component in-vivo which produces hydrogen peroxide as an enzyme product through the action of an enzyme. An in-vivo component measuring device (hereinafter also simply referred to as "measuring device") will be described with reference to the drawings. As shown in FIGS. 4 to 6, the measuring device 10 includes a display unit 11, a recording unit 12, an analysis unit 13, a detection unit 14, a power source 15, and an operation button 16 which used by a subject to operate the measurement device 10. As shown in FIG. 4, the measuring device 10 is used by installing the enzyme sensor 20 and a collecting member 300 which collects the interstitial fluid extracted from the subject in the installation part 14a.

The collecting member 300 is a member for accumulating the interstitial fluid extracted from the subject by a microinvasive interstitial fluid extraction technique. The collection member 300 includes an extraction medium capable of accumulating interstitial fluid, and a support member that supports the extraction medium and has an adhesive layer. The collection member 300 can be attached to the subject's skin by an adhesive layer. Examples of the extraction medium include, but are not limited to, gels such as polyvinyl alcohol gel and the like. According to the collecting member 300 including the gel, the tissue fluid can be extracted from the skin of the living body by hygroscopicity possessed by the gel.

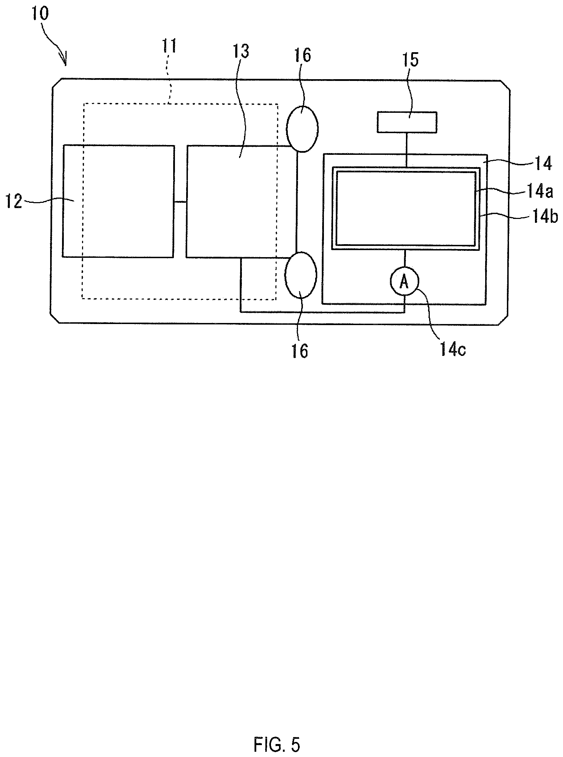

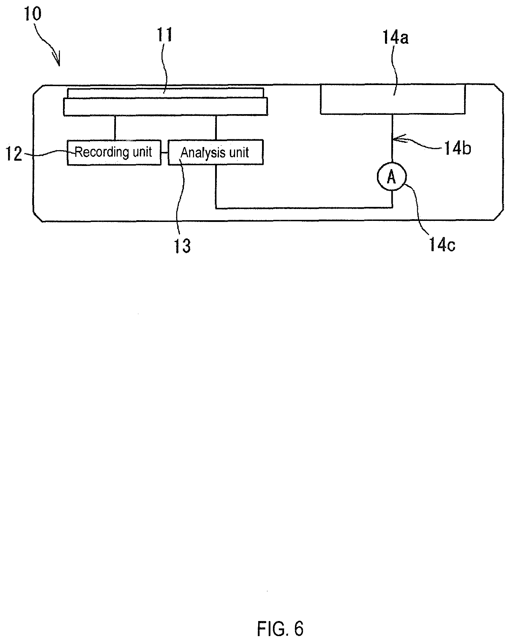

In FIGS. 5 and 6, the display unit 11 has a function of displaying the measurement result of the analysis unit 13 and the data recorded in the recording unit 12. The recording unit 12 has a function of storing past data, and a function of temporarily storing the measurement result. The analysis unit 13 has a function of calculating a concentration of an in-vivo component in a tissue fluid, specifically, a glucose concentration based on the information output from the detection unit 14. The detecting unit 14 includes an installation part 14a, an electric circuit 14b, and an ammeter 14c. The installation part 14a is a concave recessed portion capable of housing the enzyme sensor 20. The electric circuit 14b includes three terminals (not shown) exposed in the installation portion 14a. The electric circuit 14b is connected to the enzyme sensor 20 via the three terminals. The electric circuit 14b also is connected to the ammeter 14c. The ammeter 14c has a function of measuring the current generated in the enzyme sensor 20 flowing through the electric circuit 14b. The operation button 16 is provided for performing an operation such as switching the display of the display unit 11.

Note that the measuring device 10 can also be used for obtaining component information on the amount of in-vivo components contained in blood or other bodily fluids instead of interstitial fluid. In the measuring apparatus 10, body fluid also may be directly supplied to the surface of the working electrode 30 of the enzyme sensor 20. In this case, the surface of the working electrode 30 can be used as a body fluid receiving part. The enzyme sensor 20 also may be incorporated directly under the skin of the subject so that component information on the amount of in-vivo component may be acquired continuously without extracting body fluids.

When using the measuring apparatus 10, the amount of the in-vivo component contained in the body fluid can be measured by, for example, a method including the following steps. (I) A step of setting the enzyme sensor 20 in the installation part 14a of the measuring apparatus 10; (II) a step of supplying body fluid onto the working electrode 30 of the enzyme sensor 20 in the setting section 14a; (III) a step of applying a predetermined voltage to the working electrode 30 with reference to the reference electrode 50 of the enzyme sensor 20 as a reference; (IV) a step of measuring the current flowing between the working electrode 30 and the counter electrode 40; and (V) a step of calculating the in vivo component amount contained in the body fluid based on the measured current.

In step (III), the voltage applied to the working electrode 30 can be appropriately determined according to the type of in-vivo component, the purpose of measuring the amount of in-vivo component and the like. When the in-vivo component is glucose, the voltage applied to the working electrode 30 is preferably 0.25 to 0.35 V from the viewpoint of suppressing interference due to interfering substances and measuring the amount of glucose with high sensitivity.

In the step (IV), for example, a chronoamperometry method and the like can be used as a method for measuring the current, but there is no particular limitation.

In step (V), the calculation of the in-vivo component amount contained in the body fluid is performed using a calibration curve of a current value prepared in advance based on the current value when a standard solution having a known amount of in-vivo component is used. When the in-vivo component is glucose, other components contained in the living body such as sodium ion and potassium ion are also measured at the same time for in-vivo components other than glucose, and the amount of glucose contained in the body fluid can be calculated by calculating the ratio of the signal obtained for glucose and the signals obtained for in-vivo components other than glucose.

Method for Producing Hydrogen Peroxide Electrode

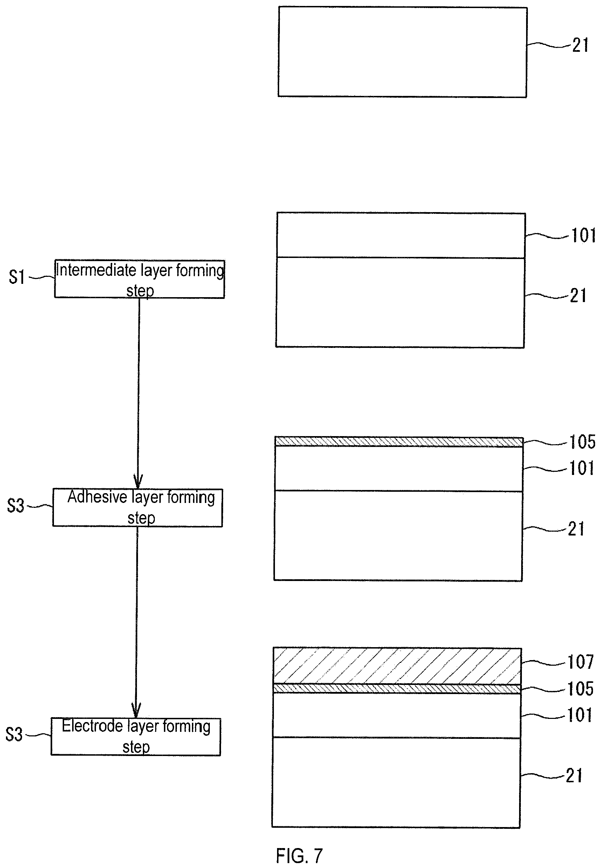

The hydrogen peroxide electrode 100 can be manufactured by a method including (A) a step of forming an intermediate layer 101 containing a hydrophilic polymer substance 111 and particles 112 on one surface of a substrate 21, and (B) a step of forming an electrode layer 107 on the surface of the intermediate layer 101. The procedure of the manufacturing method will be described hereinafter with reference to the drawings. Step S1 in FIG. 7 corresponds to the aforementioned step (A). Steps S2 and S3 in FIG. 7 correspond to the aforementioned step (B).

In step S 1, an intermediate layer 101 is formed on one surface of the substrate 21. The intermediate layer 101 can be formed by a method corresponding to the structure of the intermediate layer 101.

When the intermediate layer 101 has the single layer structure shown in FIG. 2A, first, a mixed solution containing the hydrophilic polymer substance 111 and the particles 112 is dripped on one surface of the substrate 21. The amount of the mixed solution to be dripped, the concentration of the hydrophilic polymer substance 111 in the mixed solution, and the concentration of the particles 112 in the mixed solution can be appropriately determined according to the thickness of the intermediate layer 101 and the like. Usually, the concentration of the hydrophilic polymer substance 111 in the mixed solution is preferably 1 to 50% by mass, more preferably 3 to 30% by mass, and most preferably 3 to 20% by mass. Usually, the concentration of the particles 112 in the mixed solution is preferably from 0.1 to 100% by mass, more preferably from 0.5 to 50% by mass, and most preferably from 0.5 to 30% by mass. Next, the substrate 21 is rotated using a spin coater to remove excess liquid. As a result, a coating film containing the hydrophilic polymer substance 111 and the particles 112 is formed on the substrate 21. The number of revolutions and the time of the substrate 21 by the spin coater can be appropriately determined according to the thickness of the intermediate layer 101 and the like. Next, the substrate 21 is heated to dry the coating film. The intermediate layer 101 which is the single layer 102 containing the hydrophilic polymer substance 111 and the particles 112 is formed in this way.

When the intermediate layer 101 has the two-layer structure shown in FIG. 2B, a solution containing the hydrophilic polymer substance 111 is dripped on one surface of the substrate 21, on one surface of the substrate 21. The amount of the solution to be dripped and the concentration of the hydrophilic polymer substance 111 in the solution can be appropriately determined according to the thickness of the first intermediate layer 103 or the like. Usually, the concentration of the hydrophilic polymer substance 111 in the solution is preferably 1 to 50% by mass, more preferably 3 to 30% by mass, and most preferably 3 to 20% by mass. Next, the substrate 21 is rotated using a spin coater to remove excess liquid. As a result, a coating film containing the hydrophilic polymer substance 111 is formed on the substrate 21. Next, the substrate 21 is heated to dry the coating film. The first intermediate layer 103 containing the hydrophilic polymer substance 111 is formed in this way. Next, a solution containing the particles 112 is dripped onto one surface of the substrate 21. The amount of the solution to be dripped and the concentration of the particles 112 in the solution can be appropriately determined according to the thickness of the second intermediate layer 104 and the like. Usually, the concentration of the particles 112 in the solution is preferably from 0.1 to 100% by mass, more preferably from 0.5 to 50% by mass, and most preferably from 0.5 to 30% by mass. Next, the substrate 21 is rotated using a spin coater to remove excess liquid. As a result, a coating film containing the particles 112 is formed on the substrate 21. Next, the substrate 21 is heated to dry the coating film. The second intermediate layer 104 containing particles 112 is formed in this way. In addition to the above-described method of forming the intermediate layer 101 having a two-layer structure, a method of forming the first intermediate layer 103 and subsequently immersing the first intermediate layer 103 of the substrate 21 in a solution containing the particles 112 may be used inasmuch as the method is not particularly limited.

Returning to step S2 in FIG. 7, an adhesive layer 105 is formed on the surface of the intermediate layer 101. When the adhesive layer 105 is a titanium layer, examples of a method for forming the adhesive layer 105 include, but are not limited to, a sputtering method, a vacuum vapor deposition method, an electroless plating method, an electrolytic plating method, and the like.

Next, in step S3, the electrode layer 107 is formed on the surface of the adhesive layer 105. Examples of a method for forming the electrode layer 107 include, but are not limited to, a sputtering method, a vacuum evaporation method, an electroless plating method, an electrolytic plating method, and the like.

Method of Producing Enzyme Sensor

The enzyme sensor 20 can be produced by immobilizing an enzyme on the surface of the electrode layer 107 of the hydrogen peroxide electrode 100 to form the enzyme layer 110. The fixation of the enzyme to the surface of the electrode layer 107 can be carried out by, for example, the following methods. Method (a-1): a method of applying a solution containing an enzyme on the surface of the electrode layer 107 of the hydrogen peroxide electrode 100; method (a-2): a method of immersing the surface of the electrode layer 107 of the hydrogen peroxide electrode 100 in a solution containing the enzyme; method (a-3): a method of introducing a functional group to the surface of the electrode layer 107 of the hydrogen peroxide electrode 100 to immobilize the enzyme via this functional group.

Examples

Description of Abbreviations

PET: polyethylene terephthalate
PBS: phosphate buffered saline
PVA: polyvinyl alcohol
PS: polystyrene

Interference substances examined for the structure and influence of the glucose sensors of Examples and Comparative Examples are shown in Table 1.

Table 1

| | Ex. 1 | Ex. 2 | Ex.3 | Ex. 4 | C. Ex. 1 | C. Ex. 2 | C. Ex. 3 | C. Ex. 4 |
|---|---|---|---|---|---|---|---|---|
| Particle | Aluminah ydrate particles | PS beads | Mesoporo us silica | PS beads | -- | -- | PS beads | -- |
| Ave Dia. | 100 nm | 500 nm | -- | 1.5 $\mu$m | | | 500 nm | |
| Hydrophi c polymer | Partially saponified PVA; silinized modified PVA | PVA | PVA | PVA | -- | -- | -- | PVA |
| Intermediate layer form | Mixed layer | Two layers | Two layers | | | | | |
| Adhesive layer titanium layer | 5 nm | 10 nm | 5 nm | 10 nm | 5 nm | 10 nm | 10 nm | 5 nm |
| Electrode layer | 100 nm | 200 nm | 100 nm | 200 nm | 100 nm | 200 nm | 200 nm | 100 nm |

(continued)

| | Ex. 1 | Ex. 2 | Ex.3 | Ex. 4 | C. Ex. 1 | C. Ex. 2 | C. Ex. 3 | C. Ex. 4 |
|---|---|---|---|---|---|---|---|---|
| Interferin g substance | Acetamin ophen | Acetamin ophen | Acetamin ophen; ascorbic acid | Acetamin ophen; ascorbic acid | Acetamin ophen | Acetamin ophen; ascorbic acid | | |

Example 1

Hereinafter, the influence of the presence or absence of an intermediate layer which is a mixed layer of alumina hydrate particles and a hydrophilic polymer substance on the detection of glucose in the presence of interfering substances, as shown by Example 1 and Comparative Examples 1 and 2, is examined. In Example 1, a mixed layer of alumina hydrate particles and a hydrophilic polymeric substance was used as the intermediate layer.

(1) Intermediate Layer Formation

A particle containing liquid was obtained by adding 100 g of alumina hydrate particles (average particle diameter: 100 nm), 15 g of partially saponified PVA, and 2 g of silanized modified PVA to 614 g of ion-exchanged water heated to 95°C and agitating the solution.
Then, 0.3 g of the obtained particle-containing liquid was applied to one surface of a PET film (length: 3 cm, width: 3 cm, thickness: 75 $\mu$m) as a substrate. The PET film coated with the particle-containing liquid was rotated at 750 rpm for 10 seconds by a spin coater to remove excess liquid to form a coating film. Thereafter, the PET film having the coating film was heated at 110°C for 3 minutes to obtain an intermediate layer ("mixed layer" in Table 1) containing PVA which is a hydrophilic polymer substance and fixed alumina hydrate particles laminated on a film.

(2) Electrode Layer Formation

A titanium layer (thickness: about 5 nm) was formed on the intermediate layer obtained in Example 1 (1) by the sputtering method. Next, in order to obtain the pattern shown in FIG. 3, an electrode layer (diameter: 8 mm, thickness: about 100 nm) for a working electrode containing platinum, and an electrode layer for a counter electrode containing platinum (thickness: about 100 nm) were formed on the titanium layer by sputtering method. A silver/silver chloride ink also was coated on the titanium layer so as to have the pattern shown in FIG. 3, and dried to form a reference electrode made of silver/silver chloride. The electrode substrate was obtained in this way.

(3) Enzyme Layer Formation

A 10 $\mu$L enzyme solution (0.25 % by mass glutaraldehyde, 42.984 mg/dL bovine serum albumin, 1.5225 U/ml glucose oxidase, and 0.5 U/ml mutarotase in PBS) was dripped on the working electrode of the electrode substrate obtained in Example 1 (2). Next, the electrode substrate was allowed to stand in an environment kept at 25°C at a relative humidity of 30%, to dry the enzyme solution. An enzyme layer was formed on the working electrode to obtain a glucose sensor in this way.

(4) Glucose Solution and Acetaminophen Solution Preparation

A glucose solution having glucose concentrations of 0.05 mg/dL, 0.2 mg/dL, or 0.8 mg/dL was obtained by diluting a glucose standard solution (manufactured by SYSMEX CORPORATION, trade name: glucose standard solution) with PBS. In addition, by diluting acetaminophen with PBS, an acetaminophen solution having acetaminophen concentration of 0.0025 m/dL, 0.01 mg/dL, or 0.04 mg/dL was obtained.

(5) Hydrogel Preparation

PVA hydrogel was obtained by irradiating an electron beam having a dose of 25 kGr on a PVA aqueous solution A [composition: 12% (w/w) PVA, 2% (w/w) potassium chloride and 86% (w/w) water].

(6) Electrochemical Measurement

The hydrogel obtained in Example 1 (5) was pasted inside a flow cell. The glucose sensor obtained in Example 1 (3) was placed in contact with the hydrogel in the flow cell. Hydrogel and glucose sensor were immobilized with a jig of flow cell. By using a syringe pump, PBS was flowed into the flow cell, and the glucose sensor and the PBS were brought into contact with each other via the hydrogel.

The glucose sensor was connected to a potentiostat (product name: ALS832b, manufactured by BAS Inc.). Voltage was applied to the working electrode of the glucose sensor with the reference electrode as a reference. The current flowing between the working electrode and the counter electrode was measured by the chronoamperometry method.

(7) Evaluation of Applied Voltage Dependence of Glucose Detection Sensitivity

After the current value was stabilized in the electrochemical measurement of Example 1 (6), PBS, 0.05 mg/dL glucose solution, 0.2 mg/dL glucose solution, 0.8 mg/dL glucose solution and PBS were added into a flow cell at a flow rate of 0.5 mL/min for 10 minutes, and the current value was measured. The average value of the current value measured over 200 seconds after the passage of 400 seconds from the start of flowing the glucose solution of each concentration was obtained as the current value for the glucose solution of each concentration.

The applied voltage to the working electrode was changed to 0.25 V, 0.3 V, 0.4 V, or 0.5 V, and the dependence of the detection sensitivity of glucose on the applied voltage was evaluated. The detection sensitivity of glucose represented by equation (I):

$$[0074] \text{(detection sensitivity of glucose)} = \text{(amount of change of current value when glucose concentration is changed)} / \text{(amount of change of glucose concentration)} \quad (I)$$

was determined pursuant with the equation.

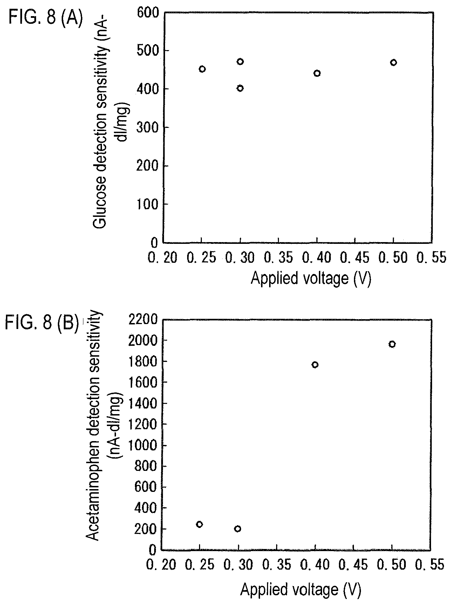

FIG. 8 (A) shows the relationship between the detection sensitivity of glucose and applied voltage when the glucose sensor of Example 1 is used.

(8) Evaluation of Applied Voltage Dependence of Acetaminophen Detection Sensitivity

After the current value was stabilized in the electrochemical measurement of Example 1 (6), PBS, 0.0025 mg/dL acetaminophen solution, 0.01 mg/dL acetaminophen solution, 0.04 mg/dL acetaminophen solution and PBS were flowed into the flow cell in this order at a flow rate of 0.5 mL/min for 10 minutes, and the current value was measured. The average value of the current value measured over 200 seconds after the passage of 400 seconds from the start of flowing acetaminophen solution of each concentration was obtained as the current value for each concentration of acetaminophen solution.

The applied voltage to the working electrode was changed to 0.25 V, 0.3 V, 0.4 V, or 0.5 V, and the dependence of the detection sensitivity of acetaminophen on the applied voltage was evaluated. The detection sensitivity of acetaminophen represented by equation (II):

$$[0079] \text{(detection sensitivity of acetaminophen)} = \text{(amount of change of current value when acetaminophen concentration is changed)} / \text{(amount of change of acetaminophen concentration)} \quad (II)$$

was determined pursuant with the equation.

The relationship between the detection sensitivity of acetaminophen and the applied voltage when the glucose sensor of Example 1 is used is shown in FIG. 8 (B).

(9) Results

From the results shown in FIG. 8 (A), it was understood that the detection sensitivity of glucose was constant regardless of the voltage applied to the working electrode. On the other hand, from the results shown in FIG. 8 (B), it was found that the detection sensitivity of acetaminophen depends on the voltage applied to the working electrode. These results suggested that glucose can be detected with high sensitivity without lowering the interference or disturbance by acetaminophen if the voltage applied to the working electrode is lowered.

Comparative Example 1 (1) Electrode Layer Formation

A titanium layer (thickness: about 5 nm) was formed on a PET film (length: 3 cm, width: 3 cm, thickness: 75 μm) substrate by a sputtering method. Next, in order to obtain the pattern shown in FIG. 3, an electrode layer (diameter: 8 mm, thickness: about 100 nm) for a working electrode containing platinum, and an electrode layer for a counter electrode containing platinum (thickness: about 100 nm) were formed on the titanium layer by sputtering method. A silver/silver chloride ink also was coated on the titanium layer so as to have the pattern shown in FIG. 3, and dried to form a reference electrode made of silver/silver chloride. The electrode substrate was obtained in this way.

(2) Enzyme Layer Formation

A glucose sensor was obtained in the same manner as in Example 1 (3), except that the electrode substrate obtained in Comparative Example 1 (1) was used.

(3) Electrochemical Measurement

The same operation as in Example 1 (6) was carried out except that the glucose sensor obtained in Comparative Example 1 (2) was used, and electrochemical measurement was carried out.

(4) Evaluation of Applied Voltage Dependence of Glucose Detection Sensitivity

The same operation as in Example 1 (7) was carried out except that the glucose sensor was used after the current value had been stabilized in the electrochemical measurement of Comparative Example 1 (3), and the applied voltage dependency of the detection sensitivity of glucose was evaluated.

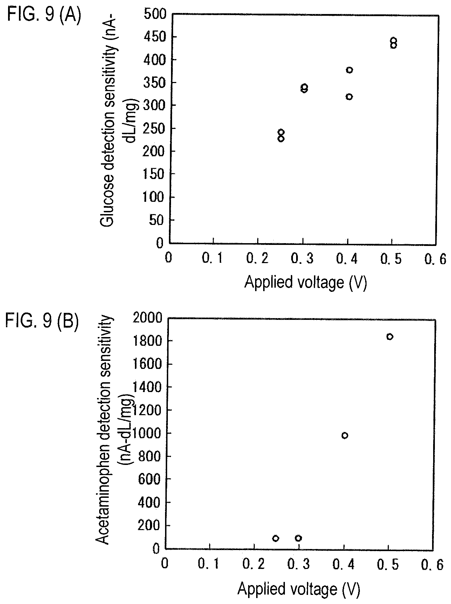

FIG. 9 (A) shows the relationship between the detection sensitivity of glucose and applied voltage when the glucose sensor of Comparative Example 1 is used.

(5) Evaluation of Applied Voltage Dependence of Acetaminophen Detection Sensitivity

The same operation as in Example 1 (8) was carried out except that the glucose sensor was used after the current value had been stabilized in the electrochemical measurement of Comparative Example 1 (3), and the applied voltage dependency of the detection sensitivity of acetaminophen was evaluated.
The relationship between the detection sensitivity of acetaminophen and the applied voltage when the glucose sensor of Example 1 is used is shown in FIG. 9 (B).

(6) Results

From the results shown in FIG. 9 (A), it was understood that the detection sensitivity of glucose increased as the voltage applied to the electrode was increased, and decreased when the voltage applied to the electrode was reduced. Therefore, it was understood that it is necessary to increase the applied voltage in order to detect glucose with high sensitivity From the results shown in FIG. 9 (B), it was also understood that the detection sensitivity of acetaminophen also depends on the voltage applied to the electrode, similarly to the detection sensitivity of glucose. From these results, it was understood that when the applied voltage was increased in order to detect glucose with high sensitivity, it was subjected to interference by acetaminophen. Therefore, according to the glucose sensor of Comparative Example 1, glucose could not be detected with high sensitivity without being affected by interfering substances such as acetaminophen. The glucose sensor of Example 1 and the glucose sensor of Comparative Example 1 differed with regard to the presence or absence of an intermediate layer. These results suggested that, since the glucose sensor of Example 1 has an intermediate layer, glucose can be detected with high sensitivity without being affected by interfering substances such as acetaminophen.

Comparative Example 2 (1) Electrode Layer Formation

An electrode substrate was obtained in the same manner as in Comparative Example 1 (1) except that the thickness of the titanium layer was about 10 nm and the thickness of the electrode layer containing platinum was about 200 nm.

(2) Enzyme Layer Formation

A glucose sensor was obtained in the same manner as in Example 1 (3), except that the electrode substrate obtained in Comparative Example 2 (1) was used.

(3) Glucose Solution and Test Solution Preparation

Glucose solutions having glucose concentrations of 0.05 mg/dL, 0.1 mg/dL, 0.2 mg/dL or 0.8 mg/dL were obtained by diluting the glucose standard solution with PBS. Acetaminophen was dissolved in a 0.1 mg/dL glucose solution so that its concentration was 0.1 mg/dL to obtain an acetaminophen-containing test solution. Ascorbic acid was dissolved in a 0.1 mg/dL glucose solution so that its concentration was 0.1 mg/dL to obtain an ascorbic acid-containing test solution.

(4) Electrochemical Measurement

The glucose sensor obtained in Comparative Example 2 (2) was connected to a potentiostat (product name: ALS832b, manufactured by BAS Inc.). Next, 400 µL of PBS was dripped onto the glucose sensor. Thereafter, voltage was applied to the working electrode of the glucose sensor with the reference electrode as a reference. The current flowing between the working electrode and the counter electrode was measured by the chronoamperometry method.

(5) Evaluation of Applied Voltage Dependence of Glucose Detection Sensitivity

After the current value was stabilized in the electrochemical measurement of Comparative Example 2 (4), 400 µL of PBS, 400 µL of 0.05 mg/dL glucose solution, 400 µL of 0.2 mg/dL glucose solution, and 400 µL of 0.8 mg/dL glucose solution and 400 µL of PBS was dropped into the glucose sensor in this order and the current value was measured for 180 seconds. The average value of the current value measured over 30 seconds after the lapse of 150 seconds after dripping the glucose solution of each concentration was obtained as the current value of the glucose solution of each concentration.

The applied voltage to the working electrode was changed to 0.25 V, 0.3 V, 0.4 V, or 0.5 V, and the dependence of the detection sensitivity of glucose on the applied voltage was evaluated. Detection sensitivity of glucose was determined according to equation (I).

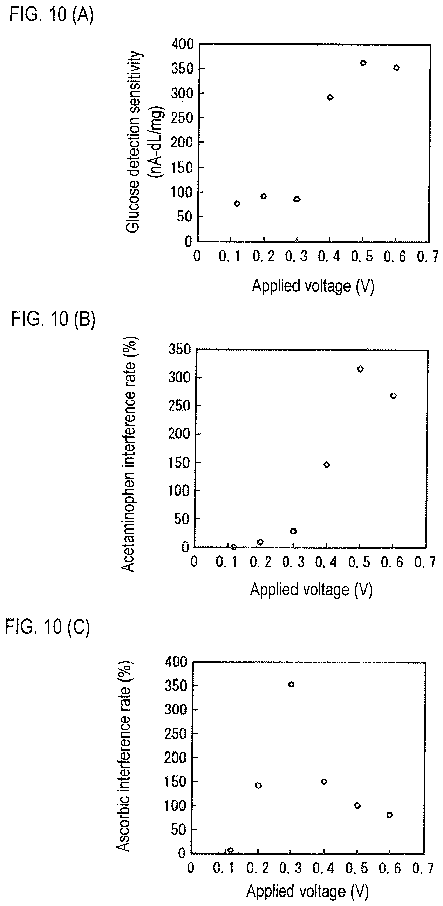

FIG. 10 (A) shows the relationship between the detection sensitivity of glucose and applied voltage when the glucose sensor of Comparative Example 2 is used.

(6) Evaluation of Influence by Interfering Substances

After the current value stabilized at the time of electrochemical measurement of the Comparative Example 2 (4), 400 µL of PBS, 400 µL of 0.1 mg/dL glucose solution, 400 µL of PBS, 400 µL of acetaminophen-containing test solution, 400 µL of PBS, 400 µL of test solution containing ascorbic acid, and 400 µL of PBS in this order were dripped onto the glucose sensor, and the current value was measured for 180 seconds. The average value of the current value measured over 30 seconds after the lapse of 150 seconds after dripping each solution was obtained as the current value of each solution.

The voltage applied to the working electrode was changed to 0.12 V, 0.2 V, 0.3 V, 0.4 V, 0.5 V or 0.6 V, and the influence by the interfering substance was evaluated.

The interference rate by acetaminophen represented by equation (III):

$$\text{(Interference rate by acetaminophen)} = 100 \times ([\text{current value of acetaminophen-containing test solution}] - [\text{current value of } 0.1 \text{ mg/dL glucose solution}] / [\text{current value of } 0.1 \text{ mg/dL glucose solution}]) \quad (III)$$

was determined pursuant with the equation. Further, the interference rate by ascorbic acid expressed by equation (IV):

$$[\text{interference rate by ascorbic acid}] = 100 \times ([\text{current value of test solution containing ascorbic acid}] - [\text{current value of } 0.1 \text{ mg}/dL \text{ glucose solution}] / [\text{current value of } 0.1 \text{ mg/dL glucose solution}]) \quad (IV)$$

was determined pursuant with the equation.

The relationship between the interference rate due to acetaminophen and the applied voltage when using the glucose sensor of Comparative Example 2 is shown in FIG. 10 (B), and the interference rate by ascorbic acid and the applied voltage when using the glucose sensor of Comparative Example 2 is shown in FIG. 10 (C).

(7) Results

From the results shown in FIG. 10 (A), it was understood that the detection sensitivity of glucose increased as the voltage applied to the electrode increased, and decreased when the voltage applied to the electrode was reduced. Further, from the results shown in FIGS. 10 (B) and (C), it was found that interference was caused by acetaminophen and ascorbic acid, respectively, when the applied voltage was increased in order to detect glucose with high sensitivity. Therefore, according to the glucose sensor of Comparative Example 2, glucose could not be detected with high sensitivity without being affected by interfering substances. The glucose sensor of Example 1 and the glucose sensor of Comparative Example 2 differed with regard to the presence or absence of an intermediate layer. These results suggested that, since the glucose sensor of Example 1 has an intermediate layer, glucose can be detected with high sensitivity without being affected by interfering substances such as acetaminophen.

Example 2

Hereinafter, the influence of the presence or absence of an intermediate layer on the detection of glucose in the presence of interfering substances is examined according to Example 2 and Comparative Example 2. In Example 2, two intermediate layers configured by a layer containing a PS bead and a layer containing a hydrophilic polymer substance were used as the intermediate layer.

(1) First Intermediate Layer Formation

An aqueous PVA solution B was obtained by dissolving 2.5 g of PVA in 50 g of ion exchanged water heated to 95°C with agitation. Then, 500 $\mu$L of the obtained PVA aqueous solution B was applied to one surface of a PET film (length: 3 cm, width: 3 cm, thickness: 75 $\mu$m) as a substrate. The PET film coated with the PVA aqueous solution was rotated at 750 rpm for 10 seconds by a spin coater to remove excess liquid to form a coating film. Thereafter, the PET film having the coating film was heated at 110°C for 3 minutes to fix the first intermediate layer containing the hydrophilic polymeric substance PVA on the film. The intermediate substrate A having the first intermediate layer was obtained in this way.

(2) Second Intermediate Layer Formation

On the first intermediate layer of the intermediate substrate A obtained in Example 2 (1), 500 $\mu$L of a solution containing PS beads [average particle diameter of PS beads: 500 nm, Thermo Fisher Scientific; product name: 5050A] was dripped. The intermediate substrate A onto which the PS bead-containing solution was dripped was rotated at 300 rpm for 10 seconds by a spin coater to remove excess liquid. Thereafter, the intermediate substrate A was heated at 110°C. for 3 minutes to fix the second intermediate layer including PS beads on the first intermediate layer. The intermediate substrate B having the working electrode including the second intermediate layer was thus obtained.

(3) Electrode Layer Formation

An electrode substrate was obtained performing the same operations as Example 1 (2) except that the intermediate substrate B obtained in Example 2 (2) was used, the thickness of the titanium layer was set to about 10 nm, the thickness of the electrode layer used for the counter electrode layer was about 200 nm and the thickness of the working electrode was set to about 200 nm.

(4) Enzyme Layer Formation

A glucose sensor was obtained in the same manner as in Example 1 (3), except that the electrode substrate obtained in Comparative Example 2 (3) was used.

(5) Electrochemical Measurement

The same operation as in Comparative Example 2 (4) was performed except that the glucose sensor obtained in Example 2 (4) was used, and the current flowing between the working electrode and the counter electrode was measured.

(6) Evaluation of Applied Voltage Dependence of Glucose Detection Sensitivity

The same operation as Comparative Example 2 (5) was performed, except for using the glucose sensor after the current value was stabilized in the electrochemical measurement of Example 2 (5) and changing the voltage applied to the working electrode to 0.05 V or 0.1 V, to evaluate the dependence of the detection sensitivity of glucose upon application of a low voltage.

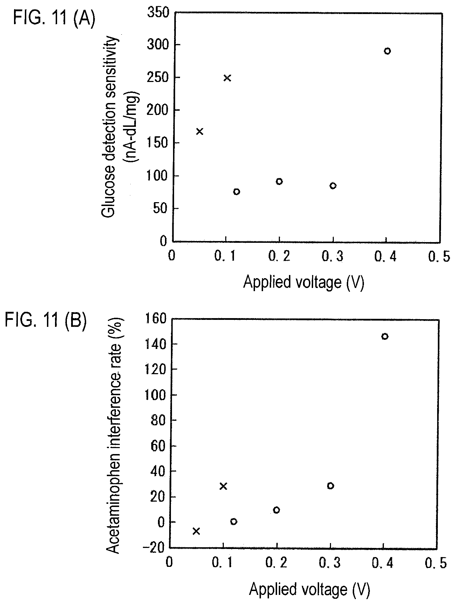

FIG. 11 (A) shows the relationship between the detection sensitivity of glucose and applied voltage when the glucose sensors of Example 2 and Comparative Example 2 are used. In the drawing, the crosses ("X") indicates the result of examining the relationship between the detection sensitivity of glucose and the applied voltage when using the glucose sensor of Example 2, and the white circles indicate the result of examining the relationship between glucose when using the glucose sensor of Comparative Example 2.

(7) Evaluation of Influence by Interfering Substances

After the current value was stabilized at the time of electrochemical measurement of Example 2 (5), 400 μL of PBS, 400 μL of 0.1 mg/dL glucose solution, 400 μL of PBS, 400 μL of acetaminophen-containing test solution, 400 μL of PBS, and 400 μL of PBS in this order were dripped onto the glucose sensor, and the current value was measured for 180 seconds. The average value of the current value measured over 30 seconds after the lapse of 150 seconds after each solution was dripped was obtained as the current value of each solution.

The voltage applied to the working electrode was changed to 0.05 V or 0.1 V and the influence by acetaminophen at the time of low voltage application was evaluated. The interference rate due to acetaminophen was determined according to equation (III). The interference rate due to ascorbic acid was determined according to equation (IV).

The relationship between the interference rate due to acetaminophen and the applied voltage when the glucose sensors of Example 2 and Comparative Example 2 are used is shown in FIG. 11 (B). In the drawing, the crosses indicate the result of examining the relationship between the detection sensitivity of glucose and the applied voltage when using the glucose sensor of Example 2, and the white circles indicate the result of examining the relationship between glucose when using the glucose sensor of Comparative Example 2.

(8) Results

It was understood from the results shown in FIG. 11 (A), when the applied voltage to the working electrode is 0.25 V or less, the detection sensitivity of glucose when using the glucose sensor of Example 2 is higher than the detection sensitivity of glucose when using the glucose sensor of Comparative Example 2. It was also understood from the results shown in FIG. 11 (B), when the applied voltage to the working electrode is 0.25 V or less, the interference rate by acetaminophen when using the glucose sensor of Example 2 is less than or equal to the interference rate by acetaminophen when glucose sensor of Comparative Example 2 was used.

The glucose sensor of Example 2 was provided with a working electrode including an intermediate layer containing PS beads between the substrate and the electrode layer. In contrast, the glucose sensor of Comparative Example 2 did not have an intermediate layer. Therefore, this suggests that glucose can be detected with high sensitivity without interference from interfering substances when using a glucose sensor having a working electrode that includes the intermediate layer containing particles between the substrate and the electrode layer, and an applied voltage which is unlikely to have interference by an interfering substance such as acetaminophen.

Example 3

Hereinafter, the influence of the presence or absence of an intermediate layer on the detection of glucose in the presence of interfering substances is examined according to Example 3 and Comparative Example 2. In Example 3, two intermediate layers configured by a layer containing mesoporous silica and a layer containing a hydrophilic polymer substance were used as the intermediate layer.

(1) First Intermediate Layer Formation

The same operation as in Example 2(1) was carried out to obtain an intermediate substrate A having a first intermediate layer.

(2) Second Intermediate Layer Formation

A 500 μL volume of an aqueous solution containing 1% (w/w) mesoporous silica (trade name: MCM-41, manufactured by Sigma-Aldrich Corp.) was dripped onto the first intermediate layer of the intermediate substrate A obtained in Example 3 (1). The intermediate substrate A onto which the mesoporous silica-containing solution was dripped was rotated at 300 rpm for 10 seconds by a spin coater to remove excess liquid. Thereafter, the intermediate substrate A was heated at 110°C for 3 minutes to fix the second intermediate layer including mesoporous silica on the first intermediate layer. The intermediate substrate B having the second intermediate layer was obtained in this way.

(3) Electrode Layer Formation

An electrode substrate was obtained in the same manner as in Example 1 (2) except that the intermediate substrate B obtained in Example 3 (2) was used.

(4) Enzyme Layer Formation

A glucose sensor was obtained in the same manner as in Example 1 (3), except that the electrode substrate obtained in Example 3 (3) was used.

(5) Electrochemical Measurement

The same operation as in Comparative Example 2 (4) was performed except that the glucose sensor obtained in Example 3 (4) was used, and the current flowing between the working electrode and the counter electrode was measured.

(6) Evaluation of Applied Voltage Dependence of Glucose Detection Sensitivity

The same operation as Comparative Example 2 (5) was performed, except for using the glucose sensor after the current value was stabilized in the electrochemical measurement of Example 3 (5) and changing the voltage applied to the working electrode to 0.18 V, 0.25 V, 0.35 V, or 0.45 V to evaluate the dependence of the detection sensitivity of glucose upon the applied voltage.

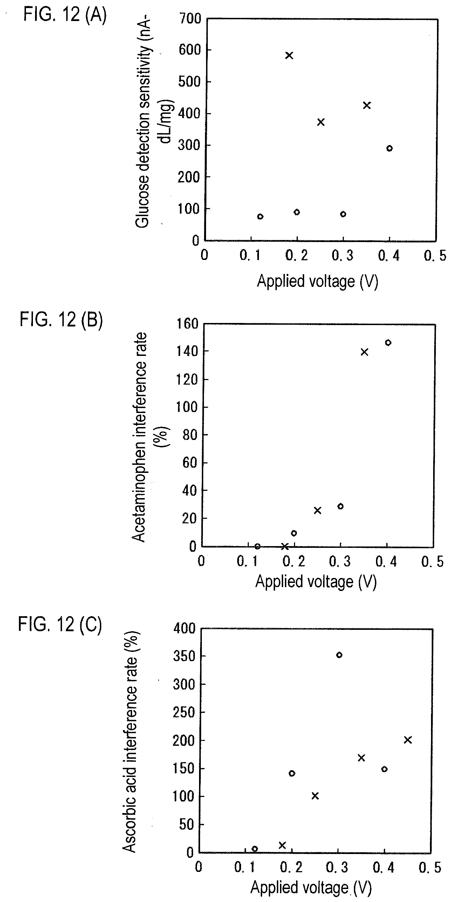

FIG. 12 (A) shows the relationship between the detection sensitivity of glucose and applied voltage when the glucose sensors of Example 3 and Comparative Example 2 are used. In the drawing, the crosses indicate the result of examining the relationship between the detection sensitivity of glucose and the applied voltage when using the glucose sensor of Example 3, and the white circles indicate the result of examining the relationship between the detection sensitivity of glucose and the applied voltage when using the glucose sensor of Comparative Example 2.

(7) Evaluation of Influence by Interfering Substances

The same operation as Comparative Example 2 (6) was performed, except for using the glucose sensor after the current value was stabilized in the electrochemical measurement of Example 3 (5) and changing the voltage applied to the working electrode to 0.18 V, 0.25 V, 0.35 V, or 0.45 V to evaluate the dependence of the detection sensitivity of glucose upon the applied voltage.
The relationship between the interference rate due to acetaminophen and the applied voltage when the glucose sensors of Example 3 and Comparative Example 2 are used is shown in FIG. 12 (B). In the drawing, the crosses indicate the result of examining the relationship between the interference rate of acetaminophen and the applied voltage when using the glucose sensor of Example 3, and the white circles indicate the result of examining the relationship between the interference rate of acetaminophen and the applied voltage when using the glucose sensor of Comparative Example 2. The relationship between the interference rate due to ascorbic acid and the applied voltage when the glucose sensors of Example 3 and Comparative Example 2 are used is shown in FIG. 12 (C). In the drawing, the crosses indicate the result of examining the relationship between the interference rate of ascorbic acid and the applied voltage when using the glucose sensor of Example 3, and the white circles indicate the result of examining the relationship between the interference rate of ascorbic acid and the applied voltage when using the glucose sensor of Comparative Example 2.

(8) Results

It was understood from the results shown in FIG. 12 (A), when the applied voltage to the working electrode is 0.4 V or less, the detection sensitivity of glucose when using the glucose sensor of Example 3 is higher than the detection sensitivity of glucose when using the glucose sensor of Comparative Example 2. It also was understood from the results shown in FIG. 12 (B), when the applied voltage was 0.4 V or less, the interference rate due to acetaminophen using the glucose sensor of Example 3 was equal to or lower than the interference rate due to acetaminophen when using the glucose sensor of Comparative Example 2. It was also understood from the results shown in FIG. 12 (C), when the applied voltage to the working electrode is 0.4 V or less, the interference rate by ascorbic acid when using the glucose sensor of Example 3 is equal to or less than the interference rate by ascorbic acid when using the glucose sensor of Comparative Example 2.

The glucose sensor of Example 3 provides a working electrode including an intermediate layer containing mesoporous silica particle which is a particle made of a porous material instead of the particles used in the glucose sensors of Examples 1 and 2 between the substrate and the electrode layer. In contrast, the glucose sensor of Comparative Example 2 did not have an intermediate layer. Accordingly, the results suggest that glucose can be detected with high sensitivity without interference from interfering substances when using a glucose sensor having a working electrode that includes the intermediate layer containing particles between the substrate and the electrode layer, and an applied voltage which is unlikely to have interference by an interfering substance such as acetaminophen and ascorbic acid.

Example 4

Hereinafter, the influence of the presence or absence of an intermediate layer on the detection of glucose in the presence of interfering substances is examined according to Example 4 and Comparative Example 2. In Example 4, as an intermediate layer, two intermediate layers of a layer containing PS beads having a larger average particle size than the PS beads used in Example 2 and a layer containing a hydrophilic polymer substance are used.

(1) First Intermediate Layer Formation

The same operation as in Example 2 (1) was carried out to obtain an intermediate substrate A having a first intermediate layer.

(2) Second Intermediate Layer Formation

The same operation was carried out as in Example 2 (2) to obtain the intermediate substrate B configuring the second intermediate layer except that the intermediate substrate A obtained in Example 4 (1) was used and that PS beads-containing solution [average particle diameter of PS beads: 1.5 $\mu$m, manufactured by Thermo Fisher Scientific Co., Ltd. , trade name: 5153A] was used.

(3) Electrode Layer Formation

An electrode substrate was obtained in the same manner as in Example 2 (3) except that the intermediate substrate B obtained in Example 4 (2) was used.

(4) Enzyme Layer Formation

A glucose sensor was obtained in the same manner as in Example 1 (3), except that the electrode substrate obtained in Example 4 (3) was used.

(5) Electrochemical Measurement

The same operation as in Comparative Example 2 (4) was performed except that the glucose sensor obtained in Example 4 (4) was used, and the current flowing between the working electrode and the counter electrode was measured.

(6) Evaluation of Applied Voltage Dependence of Glucose Detection Sensitivity

The same operation as Comparative Example 2 (5) was performed, except for using the glucose sensor after the current value was stabilized in the electrochemical measurement of Example 4 (5) and changing the voltage applied to the working electrode to 0.2 V or 0.25 V to evaluate the dependence of the detection sensitivity of glucose upon the applied voltage.

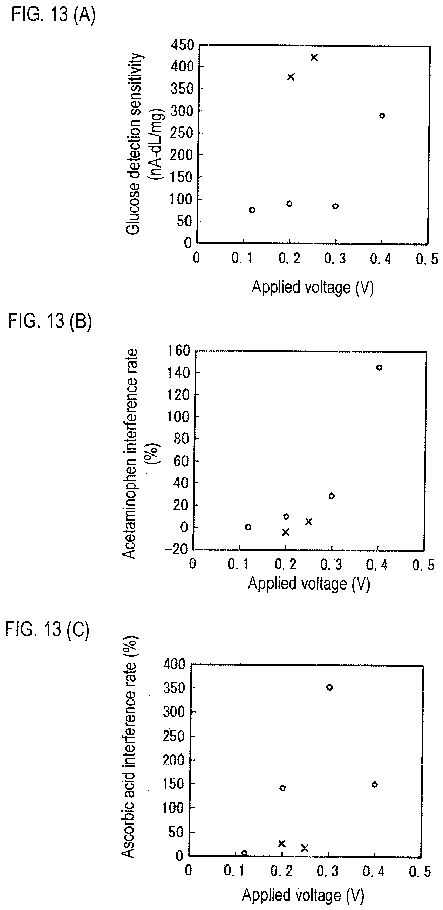

FIG. 13 (A) shows the relationship between the detection sensitivity of glucose and applied voltage when the glucose sensors of Example 4 and Comparative Example 2 are used. In the drawing, the crosses indicate the result of examining the relationship between the detection sensitivity of glucose and the applied voltage when using the glucose sensor of Example 4, and the white circles indicate the result of examining the relationship between the detection sensitivity of glucose and the applied voltage when using the glucose sensor of Comparative Example 2.

(7) Evaluation of Influence by Interfering Substances

The same operation as Comparative Example 2 (6) was performed, except for using the glucose sensor after the current value was stabilized in the electrochemical measurement of Example 4 (5) and changing the voltage applied to the working electrode to 0.2 V or 0.25 V to evaluate the influence due to acetaminophen and the influence due to ascorbic acid.

The relationship between the interference rate due to acetaminophen and the applied voltage when the glucose sensors of Example 4 and Comparative Example 2 are used is shown in FIG. 13 (B). In the drawing, the crosses indicate the result of examining the relationship between the interference rate of acetaminophen and the applied voltage when using the glucose sensor of Example 4, and the white circles indicate the result of examining the relationship between the interference rate of acetaminophen and the applied voltage when using the glucose sensor of Comparative Example 2. The relationship between the interference rate due to ascorbic acid and the applied voltage when the glucose sensors of Example 4 and Comparative Example 2 are used is shown in FIG. 13 (C). n the drawing, the crosses indicate the result of examining the relationship between the interference rate of ascorbic acid and the applied voltage when using the glucose sensor of Example 4, and the white circles indicate the result of examining the relationship between the interference rate of ascorbic acid and the applied voltage when using the glucose sensor of Comparative Example 2.

(8) Results

It was understood from the results shown in FIG. 13 (A), when the applied voltage to the working electrode is 0.4 V or less, the detection sensitivity of glucose when using the glucose sensor of Example 4 is higher than the detection sensitivity of glucose when using the glucose sensor of Comparative Example 2. It was also understood from the results shown in FIG. 13 (B), when the applied voltage to the working electrode is 0.4 V or less, the interference rate by acetaminophen when using the glucose sensor of Example 4 is less than or equal to the interference rate by acetaminophen when glucose sensor of Comparative Example 2 was used. It also was understood from the results shown in FIG. 13 (C), when the applied voltage to the working electrode is 0.4 V or less, the interference rate by ascorbic acid when using the glucose sensor of Example 4 is equal to or less than the interference rate by ascorbic acid when using the glucose sensor of Comparative Example 2.

The glucose sensor of Example 4 was provided with a working electrode including an intermediate layer containing PS beads between the substrate and the electrode layer. In contrast, the glucose sensor of Comparative Example 2 did not have an intermediate layer. Therefore, this suggests that glucose can be detected with high sensitivity without interference from interfering substances when using a glucose sensor having a working electrode that includes the intermediate layer containing particles between the substrate and the electrode layer, and an applied voltage which is unlikely to have interference by an interfering substance such as acetaminophen.

Comparative Example 3

In Comparative Example 3, the influence of using a layer of only PS beads for detection of glucose in the presence of interfering substances was investigated.

(1) A volume of 500 $\mu$L of PS bead-containing solution [average particle diameter of beads made by PS: 500 nm, 500 $\mu$L of "Thermo Fisher Scientific", product name: 5050 A) was dripped on one surface of a PET film (length: 3 cm, width: 3 cm, thickness: 75 $\mu$m) used is a substrate for forming a particle layer. The film onto which the PS bead-containing solution was dripped was rotated at 300 rpm for 10 seconds by a spin coater to remove excess liquid. Thereafter, the film was heated at 110°C for 3 minutes to fix a particle layer containing PS beads on the film.

(2) Electrode Layer Formation

A titanium layer (thickness: about 10 nm) was formed on the particle layer obtained in Comparative Example 3 (1) by the sputtering method. Next, in order to obtain the pattern shown in FIG. 3, an electrode layer (diameter: 8 mm, thickness: about 200 nm) for a working electrode containing platinum, and an electrode layer for a counter electrode containing platinum (thickness: about 100 nm) containing platinum were formed on a titanium layer by sputtering method. A silver/silver chloride ink also was coated on the titanium layer so as to have the pattern shown in FIG. 3, and dried to form a reference electrode made of silver/silver chloride. The electrode substrate was obtained in this way.

(3) Electrochemical Measurement

The same operation as in Comparative Example 2 (4) was performed except that the electrode substrate obtained in Example 3 (2) was used, and the current flowing between the working electrode and the counter electrode was measured.

(4) Results

In the electrode obtained in Comparative Example 3, when a voltage was applied to the electrode after PBS was dripped, the electrode layer and the particle layer peeled off and electrochemical measurement could not be performed. Therefore, it was suggested that electrochemical measurement can not be performed merely by providing a layer containing particles between the substrate and the electrode layer. The glucose sensor of Example 4 and the glucose sensor of Comparative Example 3 differed in the presence or absence of a hydrophilic polymeric substance between the substrate and the electrode. These results suggested that, since the glucose sensor of Example 4 has the intermediate layer containing the hydrophilic polymer substance and the particles, glucose can be detected with high sensitivity without being affected by interfering substances such as acetaminophen.

Comparative Example 4

Hereinafter, in Comparative Example 4, the influence of using a layer of only a hydrophilic polymer substance for detection of glucose in the presence of an interfering substance was examined.

(1) Hydrophilic Polymer Layer Formation

A volume of 500 μL PVA aqueous solution B was applied to one surface of a PET film (length: 3 cm, width: 3 cm, thickness: 75 μm) as a substrate. The PET film coated with the PVA aqueous solution was rotated at 750 rpm for 10 seconds by a spin coater to remove excess liquid to form a coating film. Thereafter, the PET film having the coating film was heated at 110°C for 3 minutes to fix the hydrophilic polymer PVA on the film.

(2) Electrode Layer Formation

A titanium layer (thickness: about 5 nm) was formed on the hydrophilic polymer layer formed in Comparative Example 4 (1) by a sputtering method. Next, in order to obtain the pattern shown in FIG. 3, an electrode layer (diameter: 8 mm, thickness: about 100 nm) for a working electrode containing platinum, and an electrode layer for a counter electrode containing platinum (thickness: about 200 nm) containing platinum were formed on a titanium layer by sputtering method. A silver/silver chloride ink also was coated on the titanium layer so as to have the pattern shown in FIG. 3, and dried to form a reference electrode made of silver/silver chloride. The electrode substrate was obtained in this way.

(3) Enzyme Layer Formation

A glucose sensor was obtained in the same manner as in Example 1 (3), except that the electrode substrate obtained in Comparative Example 4 (2) was used.

(4) Electrochemical Measurement

The same operation as in Comparative Example 2 (4) was performed except that the glucose sensor obtained in Example 4 (3) was used, and the current flowing between the working electrode and the counter electrode was measured.

(5) Evaluation of Applied Voltage Dependence of Glucose Detection Sensitivity

The same operation as in Comparative Example 2 (5) was carried out except that the glucose sensor was used after the current value had been stabilized in the electrochemical measurement of Comparative Example 4 (4), and the applied voltage dependency of the detection sensitivity of glucose was evaluated.

(6) Results

In the glucose sensor obtained in Comparative Example 4, the detection sensitivity of glucose was 32 nA/mg/dL when the applied voltage to the working electrode was 0.3 V with reference to the silver/silver chloride reference electrode. Therefore, it was understood that the detection sensitivity of glucose by the glucose sensor obtained in Comparative Example 4 was very low as compared with the detection sensitivity of glucose by the glucose sensor obtained in Examples 2 to 4 or Comparative Example 2. In the glucose sensor obtained in Comparative Example 4, the electrode layer also gradually peeled off when the glucose sensor was used. Accordingly, it was understood that the electrode of the glucose sensor obtained in Comparative Example 4 was fragile. The glucose sensor of Example 4 and the glucose sensor of Comparative Example 4 differed in the presence or absence of particles between the substrate and the electrode. These results suggested that, since the glucose sensor of Example 4 has the intermediate layer containing the hydrophilic polymer substance and the particles, glucose can be detected with high sensitivity without being affected by interfering substances such as acetaminophen.

Example 1 (a) First Intermediate Layer Formation

A volume of 500 μL PVA aqueous solution B was applied to one surface of a PET film (length: 3 cm, width: 3 cm, thickness: 75 μm) as a substrate. The PET film coated with the PVA aqueous solution was rotated for 10 seconds while changing the number of revolutions from 0 rpm to 300 rpm by a spin coater, and 15 seconds while changing the number of revolutions from 750 rpm to 0 rpm to remove excess liquid to form a coating film. Thereafter, the PET film having the coating film was heated at 120°C for 10 minutes to fix the first intermediate layer containing the hydrophilic polymeric substance PVA on the film. The intermediate substrate A having the first intermediate layer was obtained in this way.

(2) Second Intermediate Layer Formation

A volume of 400 μL solution containing acrylic beads having an average particle diameter of 0.1 μm, 0.5 μm, 1.5 μm or 19 μm was dripped on the first intermediate layer of the intermediate substrate A obtained in Example 1 (1). The intermediate substrate A onto which the acrylic bead-containing solution was dripped was rotated at 300 rpm for 10 seconds, and 750 rpm for 15 seconds, by a spin coater to remove excess liquid. Thereafter, the intermediate substrate A was heated at 120°C for 10 minutes to fix the second intermediate layer including acrylic beads on the first intermediate layer. The intermediate substrate B having the second intermediate layer was obtained in this way.

(3) Electrode Layer Formation

An electrode substrate was obtained in the same manner as in Example 2 (3) except that the intermediate substrate B obtained in Example 1 (2) was used.

(4) Enzyme Layer Formation

A glucose sensor was obtained in the same manner as in Example 1 (3), except that the electrode substrate obtained in Example 1 (3) was used.

(5) Electrochemical Measurement

The same operation as in Comparative Example 2 (4) was performed except that the glucose sensor obtained in Example 1 (4) was used, and the current flowing between the working electrode and the counter electrode was measured.

(6) Evaluation of Relationship of Glucose Detection Sensitivity and Average Particle Diameter

The same operation as in Comparative Example 2 (5) was carried out except that the glucose sensor was used after the current value had been stabilized in the electrochemical measurement of Example 1 (5), and the detection sensitivity of glucose was evaluated. The relationship between the average particle diameter of the particles used for the intermediate layer and the detection sensitivity of glucose was examined, and the results are shown in Table 2.

Table 2

| Average. particle diameter | Glucose detection sensitivity |
| --- | --- |
| 0.1 | 372.4 |
| 0.5 | 271.6 |
| 1.5 | 422 |
| 19 | 0.8 |

(7) Results

From the results shown in Table 2, it was understood that when acrylic beads having an average particle diameter of 0.1 $\mu$m, 0.5 $\mu$m or 1.5 $\mu$m are used, the detection sensitivity of glucose is 271 to 422 nA/mg/dL. In contrast, it also was understood that when acrylic beads having an average particle size of 19 $\mu$m were used, the detection sensitivity of glucose was 0.8 nA/mg/dL. Accordingly, these results suggest that the average particle diameter of the particles used for the intermediate layer is preferably 0.001 to 10 $\mu$m, more preferably 0.005 to 5 $\mu$m, and most preferably 0.01 to 2 $\mu$m.

Example 2 (1) First Intermediate Layer Formation

The same operation as in Example 2(1) was carried out to obtain an intermediate substrate A.

(2) Second Intermediate Layer Formation

A volume of 500 $\mu$L of solution containing PS beads having a PS bead concentration of $1.46 \times 10^{12}$/mL or $3.65 \times 10^{12}$/mL (average particle diameter of 500 nm, manufactured by Thermo Fisher Scientific Co., Ltd., trade name: 5050 A) was dripped on the first intermediate layer of the intermediate substrate A obtained in Example 2 (1). The intermediate substrate A onto which the PS bead-containing solution was dripped was rotated at 300 rpm for 10 seconds by a spin coater to remove excess liquid. By heating the intermediate substrate A after liquid removal at 110°C for 3 minutes, the second intermediate layer containing PS beads as particles having a particle density of $8.73 \times 10^4$ particles/mm$^2$ or $2.09 \times 10^5$ particles/mm$^2$ was fixed on the first intermediate layer. An intermediate substrate B having a particle density of $8.73 \times 10^4$ particles/ mm$^2$ or $2.09 \times 10^5$ particles/mm$^2$ in the second intermediate layer was obtained in this way.

A volume of 500 $\mu$L of solution containing PS beads having a PS bead concentration of $1.46 \times 10^{13}$/mL (average particle diameter of 500 nm, manufactured by Thermo Fisher Scientific Co., Ltd., trade name: 5050A) was dripped on the first intermediate layer of the intermediate substrate A obtained in Example 2 (1). The intermediate substrate A on which PS bead-containing solution was dropped was heated at 110°C for 3 minutes to obtain a second intermediate layer containing PS beads as particles and having a particle density of $8.73 \times 10^9$ particles/mm$^2$ fixed on the first intermediate layer. An intermediate substrate B having a particle density of $8.73 \times 10^9$ particles/mm$^2$ in the second intermediate layer was obtained in this way.

A PET film lacking the first intermediate layer and the second intermediate layer was prepared, and a control substrate having a particle density of 0 was obtained.

(3) Electrode Layer Formation

An electrode substrate was obtained in the same manner as in Example 2 (3) except that the intermediate substrate B obtained in Example 2 (2) was used. An electrode layer for a working electrode containing platinum, an electrode layer for a counter electrode containing platinum, and a reference electrode layer of silver/silver chloride were formed on the surface of the control intermediate substrate obtained in Example 2 (2). The electrode substrate was obtained in this way.

(4) Particle Density Evaluation

The surface of the electrode layer for the working electrode containing platinum in each of the control electrode substrates and the electrode substrate obtained in Example 2 (3) was observed with a field emission type scanning electron microscope [trade name: JSM -7500FT], and the density of PS beads immobilized on the substrate was evaluated. As a result, it was found that electrode substrates having a density of PS beads of $8.73 \times 10^4$/mm$^2$, $2.09 \times 10^5$/mm$^2$, $7.96 \times 10^5$/mm$^2$, or $8.73 \times 10^9$/mm$^2$, and a control electrode substrate with a second intermediate layer having a PS bead density of 0 were obtained.

(5) Enzyme Layer Formation

A glucose sensor was obtained in the same manner as in Example 1 (3), except that the electrode substrate obtained in Example 2 (3) was used. A control glucose sensor was obtained in the same manner as in Example 1 (3), except that the control electrode substrate obtained in Example 2 (3) was used.

(6) Electrochemical Measurement

The same operation as in Comparative Example 2 (4) was performed except that the glucose sensor obtained in Example 2 (5) or a control glucose sensor was used, and the current flowing between the working electrode and the counter electrode was measured.

(7) Evaluation of Relationship of Glucose Detection Sensitivity and Particle Density

The same operation as in Comparative Example 2 (5) was carried out except that the glucose sensor was used after the current value had been stabilized in the electrochemical measurement of Example 2 (6) and the voltage applied to the working electrode was 0.2 V, and the detection sensitivity of glucose was evaluated. The relationship between the detection sensitivity of glucose and the particle density was examined, and the results are shown in Table 3.

Table 3

| Particle density (particles/mm$^2$) | Glucose detection sensitivity (nA/mg/dL) |
|---|---|
| 0 | 61 |
| $8.73 \times 10^4$ | 74 |
| $2.09 \times 10^5$ | 89.3 |
| $7.96 \times 10^5$ | 307.6 |
| $8.73 \times 10^9$ | 154 |

(8) Evaluation of Influence by Interfering Substances

The same operation as Comparative Example 2 (6) was carried out except for using the glucose sensor after the current value was stabilized in the electrochemical measurement of Example 2 (4) and applying a voltage of 0.2 V to the working electrode, and the influence of acetaminophen during low voltage application and the influence by ascorbic acid during application of low voltage were evaluated. The results of examining the relationship between the interference rate by acetaminophen and the particle density are shown in Table 4 and the results of examining the relationship between the interference rate by ascorbic acid and the particle density are shown in Table 5.

Table 4

| Particle density (particles/mm$^2$) | Acetaminophen interference rate (%) |
|---|---|
| 0 | Not measurable due to noise |
| $8.73 \times 10^4$ | 5.5 |
| $2.09 \times 10^5$ | 10.8 |
| $7.96 \times 10^5$ | -0.9 |
| $8.73 \times 10^9$ | 0.7 |

Table 5

| Particle density (particles/mm$^2$) | Ascorbic acid interference rate |
|---|---|
| 0 | 181 |
| $8.73 \times 10^4$ | 211 |
| $2.09 \times 10^5$ | 130 |
| $7.96 \times 10^5$ | 42 |
| $8.73 \times 10^9$ | 74 |

(9) Results

From the results shown in Table 3, it was understood that the detection sensitivity of glucose when using a glucose sensor whose particle density exceeds 0 is higher than the detection sensitivity of glucose when using a glucose sensor having a particle density of 0. From the results shown in Table 4, it also was understood that when a glucose sensor whose particle density exceeds 0 is used, the interference rate by acetaminophen is reduced. In contrast, when a glucose sensor having a particle density of 0 was used, measurement could not be performed due to noise. From the results shown in Table 5, it was understood that the interference rate due to acetaminophen when a glucose sensor having a particle density exceeding $8.73 \times 10^4$ is used was lower than the interference rate due to acetaminophen when a glucose sensor having a particle density of less than $8.73 \times 10^4$ was used. These results suggested that a particle density in the intermediate layer is preferably about $9 \times 10^4$ to $9 \times 10^9$ particles/mm$^2$.

Example 3 (1) First Intermediate Layer Formation

The same operation as in Example 2 (1) was carried out to obtain an intermediate substrate A having a first intermediate layer.

(2) Second Intermediate Layer Formation

A volume of 500 μL of solution containing PS beads having a PS bead concentration of $1.46 \times 10^{13}$/mL (average particle diameter of 500 nm, manufactured by Thermo Fisher Scientific Co., Ltd., trade name: 5050A) was dripped on the first intermediate layer of the intermediate substrate A obtained in Example 3 (1). The intermediate substrate A onto which the PS bead-containing solution was dripped was rotated at 300 rpm for 10 seconds by a spin coater to remove excess liquid. After removing the liquid, the intermediate substrate A was heated at 110°C for 3 minutes. A second intermediate layer containing PS beads as particles was fixed on the first intermediate layer in this way. An intermediate substrate B of Experiment 1 having a particle density of $7.95 \times 10^5$ particles/mm$^2$ in the second intermediate layer was obtained in this way.

A volume of 500 μL of solution containing PS beads having a PS bead concentration of $1.46 \times 10^{13}$/mL (average particle diameter of 500 nm, manufactured by Thermo Fisher Scientific Co., Ltd., trade name: 5050A) was dripped on the first intermediate layer of the intermediate substrate A obtained in Example 3 (1). The intermediate substrate A on which PS bead-containing solution was dripped was heated at 110°C for 3 minutes. A second intermediate layer containing PS beads as particles was fixed on the first intermediate layer in this way. An intermediate substrate B of Experiment 2 having a particle density of $8.79 \times 10^9$ particles/mm$^2$ in the second intermediate layer was obtained in this way.

(3) Electrode Layer Formation

The same operation as Example 2 (3) was carried out except that the intermediate substrate B obtained in Example 3 (2) was used, and an electrode substrate of Experiment 1 having a particle density of $7.95 \times 10^5$ particles/mm$^2$, and an electrode substrate of Experiment 2 having a particle density of $8.79 \times 10^9$ particles/mm$^2$ were obtained.

(4) Observation of the Working Electrode Surface

The surface of the working electrode of each of the electrode substrate of Experiment No. 1, the electrode substrate of Experiment No. 2 and the control electrode substrate obtained in Example 2 (3) was observed with an electron microscope.

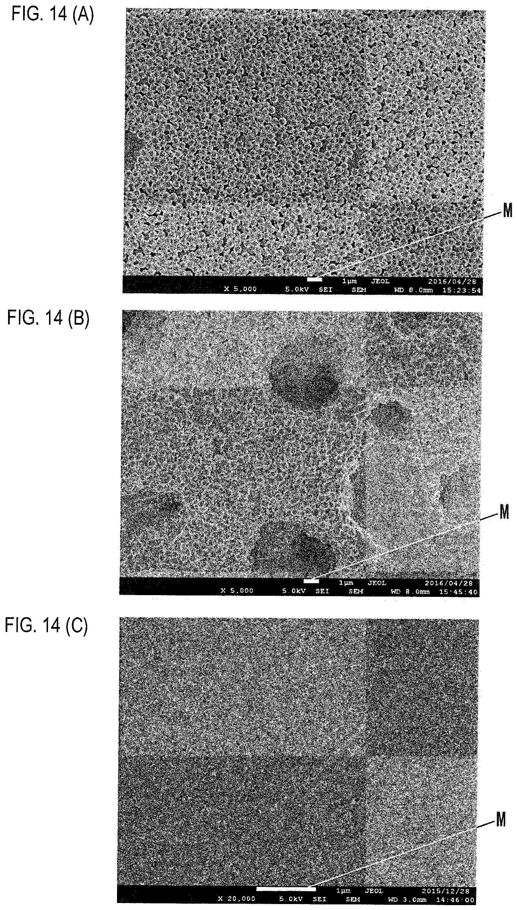

In Example 3, the result of observing the surface of the working electrode of the electrode substrate of Experiment 1 is shown in FIG. 14 (A), the result of observing the surface of the working electrode of the electrode substrate of Experiment 2 is shown in FIG. 14 (B), and the result of observing the surface of the working electrode of the control electrode substrate obtained in Example 2 (3) is shown in FIG. 14 (C). In the drawing, the scale bar M is 1 μm.

(5) Results

The results shown in FIG. 14 show the surface of the working electrodes including the particle-containing intermediate layer [refer to FIGS. 14 (A) and 14 (B)] have increased irregularities of the platinum electrode layer compared to the surface of the working electrode lacking particles [see FIG. 14 (C). That is, this suggests that irregularities are formed on the surface of the working electrode by providing an intermediate layer containing particles between the substrate and the electrode layer, and glucose can be detected with high sensitivity without interference from interfering substances due to the effect of this irregularity.

**Claims**

1. An electrode comprising:

   an intermediate layer being disposed on a substrate and including particles and a retentive substance for holding the particles; and
   an electrode layer being disposed on the intermediate layer and detecting hydrogen peroxide.

2. The electrode according to claim 1, wherein

   the electrode layer includes platinum.

3. The electrode according to claim 1 or 2, wherein

   a layer including particles and a layer including the retentive substance are laminated in the intermediate layer.

4. The electrode according to any one of claims 1 to 3, wherein

   the particles are nanoparticles or microparticles.

5. The electrode according to any one of claims 1 to 3, wherein

   the particles are porous particles.

6. The electrode according to any one of claims 1 to 5, wherein

   the particles are nonconductive particles.

7. The electrode according to any one of claims 1 to 6, wherein

   the retentive substance is a hydrophilic polymer substance.

8. The electrode according to claim 7, wherein

   the hydrophilic polymer substance is polyvinyl alcohol.

9. The electrode according to any one of claims 1 to 8, wherein

   a hydrogen peroxide selective permeation film is not provided on the electrode layer.

10. A glucose sensor comprising:

    a substrate;
    the electrode according to any one of claims 1 to 9 arranged on the substrate; and
    oxidase immobilized on a surface of the electrode.

11. A glucose sensor comprising:

    a substrate;
    the electrode according to any one of claims 1 to 9 arranged on the substrate; and
    glucose oxidase immobilized on a surface of the electrode.

12. An in-vivo component measuring device comprising:

    a detection unit for acquiring component information on an amount of an in-vivo component of a measurement target contained in a body fluid extracted from a living body;
    an analysis unit for acquiring an analysis value on the amount of the in-vivo component of the measurement target based on the component information;

wherein the detection unit comprises a working electrode comprising the electrode according to any one of claims 1 to 9 and an oxidase immobilized on a surface thereof, and a counter electrode.

13. The in-vivo component measuring device according to claim 12, wherein

the oxidase is glucose oxidase.

14. A method of manufacturing an electrode comprising:

(A) a step of forming an intermediate layer containing particles and a retentive substance for holding the particles on one surface of a substrate; and
(B) a step of forming an electrode layer for detecting hydrogen peroxide on a surface of the intermediate layer.

15. The manufacturing method according to claim 14, wherein,

in step (A), a layer containing the retentive substance is formed on the one surface of the substrate, and thereafter a layer containing the particles is formed.

Glucose      Gluconic acid

| Glucose oxidase |

$2H^+ + O_2$      $H_2O_2$

$2e^-$

110

107

30

110

107

100 { 105

101

21

FIG. 1

FIG. 2 (A)

FIG. 2 (B)

FIG. 3

FIG. 4

FIG. 5

FIG. 6

21

S1 — Intermediate layer forming step

101

21

S3 — Adhesive layer forming step

105
101

21

S3 — Electrode layer forming step

107
105
101

21

FIG. 7

FIG. 8 (A)

FIG. 8 (B)

FIG. 9 (A)

FIG. 9 (B)

FIG. 10 (A)

FIG. 10 (B)

FIG. 10 (C)

FIG. 11 (A)

FIG. 11 (B)

FIG. 12 (A)

FIG. 12 (B)

FIG. 12 (C)

FIG. 13 (A)

FIG. 13 (B)

FIG. 13 (C)

FIG. 14 (A)

FIG. 14 (B)

FIG. 14 (C)

FIG. 15

Europäisches
Patentamt

European
Patent Office

Office européen
des brevets

# EUROPEAN SEARCH REPORT

Application Number

EP 17 18 3246

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2007 187479 A (NEC CORP) 26 July 2007 (2007-07-26) * the whole document * | 1-7,9-15 | INV. G01N27/327 |
| X | US 2010/285514 A1 (CLAUSSEN JONATHAN CLAY [US] ET AL) 11 November 2010 (2010-11-11) * the whole document * | 1-5,7-15 | |
| A | GUASCITO M R ET AL: "A new amperometric nanostructured sensor for the analytical determination of hydrogen peroxide", BIOSENSORS AND BIOELECTRONICS, ELSEVIER BV, NL, vol. 24, no. 4, 1 December 2008 (2008-12-01), pages 1057-1063, XP025535199, ISSN: 0956-5663, DOI: 10.1016/J.BIOS.2008.07.048 [retrieved on 2008-08-03] * abstract * | 1-15 | |

TECHNICAL FIELDS
SEARCHED    (IPC)

G01N

The present search report has been drawn up for all claims

| Place of search | Date of completion of the search | Examiner |
|---|---|---|
| Munich | 19 October 2017 | Komenda, Peter |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

........................................................................

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 18 3246

This annex lists the patent family members relating to the patent documents cited in  the above-mentioned European search report.
The members are as contained in the European Patent Office EDP file on
The European Patent Office is in no way liable for these particulars which are merely given for the purpose of information.

19-10-2017

| Patent document cited in search report | Publication date | Patent family member(s) | Publication date |
|---|---|---|---|
| JP 2007187479    A | 26-07-2007 | NONE | |
| US 2010285514    A1 | 11-11-2010 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 63241347 A